# EUROPEAN PATENT APPLICATION

(11) **EP 4 841 638 A1**
(43) Date of publication of application: **18.11.2026**
(21) Application number: 25201637.3
(22) Date of filing: 11.09.2025
(51) Int. Cl.: G16B 15/30, G16B 35/20

(54) **A METHOD FOR EPITOPE IDENTIFICATION AND TOXICITY ASSESSMENT**

(30) Priority: 15.05.2025 DE 102025118831; 15.05.2025 DE 102025118832
(71) Applicant: BioCopy AG, 4057 Basel (CH)
(72) Inventor: AL-HASANI, Hoor, 79106 Freiburg (DE); SCHUSTER, Simon, 79241 Ihringen (DE)
(74) Representative: Hertin und Partner Rechts- und Patentanwälte PartG mbB

(57) **Abstract**

The invention relates to a computer-implemented method for predicting one or more peptides of interest binding to a protein of interest, e.g., a an antibody, antibody fragment or equivalent of, receptor, immunoglobulin or DARPin, comprising one or more of the following steps: a. providing sequence information of at least one target peptide known to bind to a protein interest, b. providing human protein sequence information, c. determining at least one peptide of interest by comparing the sequence of the at least one target peptide to the human protein sequence information provided in b., wherein the at least one peptide of interest comprises a sequence that has at least 50% sequence identity to the sequence of the least one target peptide, d. providing information of one or more single nucleotide polymorphism (SNP) present within the human genome, at least comprising the genomic location (genomic coordinates) and the polymorph amino acid of said one or more SNP, e. analyzing and annotating the one or more SNP regarding its potential position within a peptide of interest, comprising (i) determining the presence of the one or more SNP within the sequence of a peptide of interest and, if present, (ii) additionally generating the sequence of a respective peptide of interest comprising said one or more SNP, , f. predicting the binding affinity (e.g., as IC50) of the at least one peptide of interest determined in c., and optionally in e., to a HLA-molecule, thereby predicting the likelihood of the at least one peptide of interest being presented in a HLA-complex on the surface of a cell, g. combining the data obtained in steps. a.-f. and ranking, optionally using a ranking score (e.g., low, medium, high), the at least one peptide of interest regarding the likelihood of binding to the protein of interest, thereby predicting the likelihood of the one or more peptides of interest binding to a protein of interest. In embodiments peptides of interest may be presented in the context of a pHLA complex, analysed in an in vitro or in silico interaction screening regarding their binding to the at least one protein of interest.

## Description

The invention lies in the field of biology, biotechnology and biochemistry, more particular in the field of molecular biology.

The invention relates to a computer-implemented method for predicting one or more peptides of interest binding to a protein of interest, e.g., a an antibody, antibody fragment or equivalent of, receptor, immunoglobulin or DARPin, comprising one or more of the following steps: a. providing sequence information of at least one target peptide known to bind to a protein interest, b. providing human protein sequence information, c. determining at least one peptide of interest by comparing the sequence of the at least one target peptide to the human protein sequence information provided in b., wherein the at least one peptide of interest comprises a sequence that has at least 50% sequence identity to the sequence of the least one target peptide, d. providing information of one or more single nucleotide polymorphism (SNP) present within the human genome, at least comprising the genomic location (genomic coordinates) and the polymorph amino acid of said one or more SNP, e. analyzing and annotating the one or more SNP regarding its potential position within a peptide of interest, comprising (i) determining the presence of the one or more SNP within the sequence of a peptide of interest and, if present, (ii) additionally generating the sequence of a respective peptide of interest comprising said one or more SNP, , f. predicting the binding affinity (e.g., as IC50) of the at least one peptide of interest determined in c., and optionally in e., to a HLA-molecule, thereby predicting the likelihood of the at least one peptide of interest being presented in a HLA-complex on the surface of a cell, g. combining the data obtained in steps. a.-f. and ranking, optionally using a ranking score (e.g., low, medium, high), the at least one peptide of interest regarding the likelihood of binding to the protein of interest, thereby predicting the likelihood of the one or more peptides of interest binding to a protein of interest. In embodiments peptides of interest may be presented in the context of a pHLA complex, analysed in an in vitro or in silico interaction screening regarding their binding to the at least one protein of interest. In embodiments peptides of interest may be,, presented in the context of a pHLA complex, analysed in an *in vitro* or *in silico* interaction screening regarding their binding to the at least one protein of interest.

### BACKGROUND

Recent developments in adoptive cell therapy (ACT) as well as in the field of bispecific T-cell engagers (BiTE) have shown that peptide-human leukocyte antigen (pHLA)-targeting therapies represent valid strategies to treat cancer, with a first bispecific T cell receptor (TCR)-based therapy recently approved in uveal melanoma.

Peptide-HLA class I complexes are trimeric complexes that consist of a polymorphic heavy chain (alpha-chain), the light chain beta-2 microglobulin (*β*2m) and a peptide ligand, typically between 8 and 12 amino acids long and derived from cellular proteins by degradation. T cells can recognize specific peptide-HLA complexes with the TCR and initiate immune responses. Similarly, high-affinity TCR-based bispecific molecules or TCR-like antibodies bind to the respective pHLA target and trigger immune cell activity through a second binder, e.g. an anti-CD3 antibody that recruits T cells to the specific pHLA-expressing tumor cell. While such therapeutic approaches offer great potential, targeting pHLA molecules also bears the risk of off-target reactivities as experienced in some clinical trials.

Therefore, there is a clear need for comprehensive high-throughput off-target screenings for pHLA-targeting therapeutics, that can also be applied early in development to discriminate off-target profiles of different candidates. A great deal of energy has been invested in the development of soluble forms of the pHLA-reactive moiety of pHLA specific therapeutics, such as soluble T-cell receptors or TCR-like antibodies (TCRm), in order to enable interaction measurements at the molecular level.

However, high throughput methods for determining potential off-targets of a protein of interest, such as a (therapeutic) receptor, antibody, antibody fragment or DARPIN, which may cause unwanted side effect or diminish target binding, are still either limited by costs and/or the diversity and extend of the human proteome, which limits the determination of potential off-targets by *in vitro* screenings, even when performed in high-throughput.

### SUMMARY OF THE INVENTION

In light of the prior art one technical problem underlying the present invention is to provide alternative and/or improved means for selecting/predicting a set of peptides that may be analysed regarding their interaction with a protein of interest, particularly which also enable detecting a large number of successful interactions in one screen.

One problem underlying the present invention is to provide improved or alternative means for determining potential off-target binding peptides or proteins of a protein of interest, e.g., an antibody, TCR-like antibody (TCRm) or fragment thereof, or a T cell receptor or fragment.

Another problem in the prior art is that existing approaches focus on cancer mutations, lack a context for suitable peptides and/or overlook non-human peptides (e.g., of viruses found in humans).

Another problem underlying the present invention is to provide improved or alternative means for selecting the most suitable peptides and/or proteins of interest (preferably having the highest / a high likelihood of binding to a protein of interest) for performing an (preferably *in vitro*) interaction screening with a protein of interest.

These problems are solved by the features of the independent claims. Preferred embodiments of the present invention are provided by the dependent claims.

The invention relates in one aspect to a computer-implemented method for predicting one or more peptides of interest binding to a protein of interest comprising one or more of the following steps:
a. providing sequence information of at least one target peptide known to bind to a protein interest,
b. providing human protein sequence information, preferably obtained from one or more (online) data bases comprising sequence information of proteins expressed in humans, preferably comprising information on the respective amino acid sequence of said proteins, and optionally the genes encoding said proteins, their genomic location (genomic coordinates) and/or their nucleic acid sequence,
c. determining at least one peptide of interest by comparing the sequence of the at least one target peptide to the human protein sequence information provided in b., wherein the at least one peptide of interest comprises a sequence that has at least 50% sequence identity to the sequence of the least one target peptide, and optionally the same length (or optionally the same length ± 5 %, ± 15 %, ± 25 %, ± 50 %, ± 75 %) as the least one target peptide,
d. providing information on one or more single nucleotide polymorphism (SNP) present within the human genome, at least comprising the genomic location (genomic coordinates) and/or the polymorph (e.g., 'varying' or 'mutated') amino acid of said one or more SNP,
e. analyzing and annotating the one or more SNP regarding its potential position within a peptide of interest, comprising (i) determining the presence of the one or more SNP within the sequence of a peptide of interest and, if present, (ii) additionally generating the sequence of a respective peptide of interest comprising said one or more SNP, and preferably (iii) filtering the in (ii) additionally generated peptides of interest sequence(s) for a sequence identity to the sequence of the least one target peptide of ≥ 50%, preferably wherein the analysis and annotation is isoform specific,
f. predicting the binding affinity (e.g., as IC50) of the at least one peptide of interest determined in c., and optionally in e., to a HLA-molecule, thereby predicting the likelihood of the at least one peptide of interest being presented in a HLA-complex on the surface of a cell,
g. combining the data obtained in steps. a.-f. and ranking, optionally using a ranking score (e.g., low, medium, high), the at least one peptide of interest regarding the likelihood of binding to the protein of interest, preferably considering at least the sequence similarity to the at least one target peptide (e.g., employing a substitution matrix) and/or the binding affinity to a HLA-molecule determined in f., optionally further considering the physiochemical properties of the at least one peptide of interest, thereby predicting the likelihood of the one or more peptides of interest binding to a protein of interest.

In embodiments, the computer-implemented method may be for predicting the likelihood of one or more peptides of interest binding to a protein of interest, and/or for predicting one or more peptides of interest binding to a protein of interest (e.g., according to steps a-f.) comprising also predicting their likelihood of binding (e.g., according to step g).

In embodiments, any one of steps a.-g. are performed using computational means and/or employing (or assisted by) one or more (trained) machine learning (ML)-based model or artificial intelligence (Al).

In some embodiments, the present method may comprise all steps a. to g./h. in a consecutive order, in some embodiments one or more method steps may be skipped, additional steps may be added and/or the order of steps, as disclosed herein, may be amended.

In embodiments, the invention comprises a computer-implemented method for predicting or selecting (suitable or optimal) peptides of interest, which may be presented, preferably in the context of a HLA-peptide (pHLA) complex, to at least one protein of interest, e.g., a receptor, an antibody, antibody fragment or equivalent thereof, receptor, immunoglobulin or DARPin, wherein the peptides of interest, which may preferably be presented in the context of a pHLA complex, are preferably analysed in an *in vitro* or *in silico* interaction screening, preferably in an experimental interaction/binding screening regarding their binding to the at least one protein of interest.

In embodiments the present invention relates to a computer-implemented method for predicting or selecting peptides, which may be presented, preferably in the context of a pHLA complex, to at least one protein of interest, wherein the predicted or selected peptides, preferably presented in the context of a pHLA complex, are analysed subsequently in an *in vitro* interaction screening regarding their binding to the at least one protein of interest.

In embodiments the present method comprises providing sequence and/or structural data of at least one target peptide of a protein interest, and/or providing sequence and/or structural data of at least one protein of interest, and/or providing sequence and/or structural data of at least one peptide of interest, and/or providing binding / interaction data on at least one protein of interest to one or more target peptides, and/or information regarding target peptides or binding/target motifs of the at least one protein of interest, and employing one or more (trained) machine learning (ML)-based model or artificial intelligence (AI), to predict one or more peptides of interest, preferably at least their amino acid sequence, as potential interaction partner of the at least one protein of interest and for a binding or interaction screening to the at least one protein of interest, to obtain (experimental or predicted) binding data of the at least one protein of interest to different peptides.

In embodiments the predicted or selected peptides comprise or constitute targets and/or (undesired) off-targets of a protein of interest. In preferred embodiment the predicted or selected peptides are tested or analyzed in an *in vitro* interaction assay or screening regarding their positive or negative binding to the at least one protein of interest. In embodiments the present method thereby facilitates an improved and cost and time efficient way of selecting target peptides of a protein of interest, which have a high likelihood of binding to the protein of interest, such that in vitro binding assays identifying binders of a protein of interest can be performed with a pre-selected set of likely binders, thereby avoiding unnecessary and time and cost intensive screening of non-binders. In other words, the present method preferably enables excluding non-binders of a protein of interest before an invitro screening is performed, thereby saving cost and experimental time and improving the identification of relevant binders (peptides). In embodiments, also a certain binding context may be of interest, such that peptides may be predicted or selected in light of such context, e.g., a certain part of a population having a specific genetic background, such as certain SNP-variations.

The inventors developed a comprehensive method that enables predicting, based on diverse molecular and/or genetic input data, a set of peptides that have a high likelihood of interacting with a protein of interest, thereby minimizing the number and extend of (high throughput) screenings that have to be performed for detecting as many (off target) interaction partners of a protein of interest. The present approach enables saving assay costs, resources and screening time, as only a limited number of interaction screenings has to be performed to get an overview of all, or at least most of the likely off-target binders of a protein of interest. Particularly in the context of developing a therapeutic protein of interest, e.g., a therapeutic receptor or antibody, knowing the majority of unwanted off targets of a therapeutic molecule can aid avoiding later side effects in a patient and enable the further development or changes to a therapeutic molecule to avoid such side effects due to off target binding.

The present invention provides in embodiments a workflow for peptide/epitope identification to assess toxicity and possible cross or off-target reactivity of peptides with proteins of interest. In embodiments the present invention comprises multiple computational steps of a workflow that progressively apply different filters and add annotation.

In embodiments the present invention preferably also considers SNP-variations of the human genome across a large population, thereby also providing (predicting or selecting) peptides that comprise or consider SNPs in different human populations and/or ethnicities that may lead to off-target biding of proteins of interest. Prior art methods may consider cancer or disease-associated genetic variations and mutations, but fail until today to consider the naturally occurring SNP variations of the human genome and proteome with respect to potential off-target binding of proteins of interest, e.g., when used as diagnostic or therapeutic molecules.

In embodiments, in step c. the at least one peptide of interest comprises a sequence that has at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 99% sequence identity to the sequence of the least one target peptide, or a sequence identity between 20% and 99.5%, between 30% and 95%, between 40% and 95%, between 50% and 95%, between 50% and 99%, between 60% and 95%, between 30% and 95%, between 20% and 85%, between 25% and 95%, between 35% and 95%, between 40% and 95%.

In embodiments, in step c. determining at least one peptide of interest, the at least one peptide of interest comprises the (exactly) same length (or optionally the same length ± 5 %, ± 15 %, ± 25 %, ± 50 %) as the least one target peptide. In some embodiments, the target peptide may have any length, e.g., without limitation thereto, between 5-30, 5-25, 5-20, 5-15, 8-15, or 8-12 amino acids.

In some embodiments, the at least one target peptide (sequence) of a protein of interest refers to 1, 2, 3, 4, 5, or even more target peptide(s) (sequences), in some preferred embodiments only one target peptide (sequence) is considered by the present method.

In embodiments, the present method enables assessing the risk associated with any target antigen.

In embodiments off-target peptides or peptides with cross reactivity (peptides likely showing undesired interactions with a protein of interest) are first identified based on sequence similarity to a target sequence (e.g., a known target binding sequence, a sequence of a target peptide or target motif of a protein of interest), followed by a systematic evaluation using multi-layered analytical frameworks.

In embodiments, the list of potential off-target peptides or peptides with cross reactivity towards a protein of interest is quantified or supplemented by incorporation of single nucleotide polymorphism (SNPs), preferably in an early stage of the pipeline. In embodiments, relevant SNPs may be particularly present in a specific targeted population (a population of interest). The consideration of such SNPs may improve the prediction or selection of target peptides that may be analyzed *in vitro* regarding their (undesired) binding to the protein of interest, thereby enhancing the assessment of specificity of the protein of interest, which can help to reduce the overall risk of cross reactivity of the protein of interest.

In embodiments, in step d. the genomic coordinates of each peptide of interest, which were preferably extracted in step b. and/or c. are used to select and extract any SNPs found within the respective genomic coordinates of a protein comprising said peptide of interest. In embodiments, the SNPs may be subjected to filtering, e.g., according to the population, quality and/or allele frequency (> 0.1 or > 0.5-0.01 or similar). In embodiments, filtering for quality comprise selecting only experimentally and/or validated SNPs for further consideration/analysis, and discarding/ignoring merely predicted SNPs, or SNPs that only partially pass other filters.

In embodiments, NetMHCpan is used in step e. for predicting the binding of peptides of interest to MHC (HLA) class I molecules.

In embodiments the present method comprises further step h. selecting one or more of the highest ranking (e.g., when a 3-scale ranking (e.g., low, medium/moderate/intermediate) is performed, a subsequent screening may be performed of peptides of interest having a high or moderate rank; and/or of the at least 10, 20, 30 ,40 ,50, 60, 70, 80, 90, 100, 125, 150, 175, 200, or even more peptides of interest with the highest ranking) peptides of interest predicted in g. and performing an *in vitro* and/or *in silico* interaction screening to validate the predicted binding of the one or more peptides of interest to the protein of interest determined according to the method of the present invention.

In embodiments, the at least one peptide of interest is presented in the interaction screening within a HLA-peptide (pHLA) complex.

In embodiments, in step f. predicting the binding affinity (e.g., as IC50) of the at least one peptide of interest to a HLA-molecule, comprises predicting the IC50 between the peptide of interest and a HLA-molecule, preferably wherein a threshold is applied to classify binding. In embodiment, the binding affinity (IC50) of the at least one peptide of interest to a HLA-molecule is classified by a threshold determined as follows: IC50 values <50 = strong binder, 50 ≤ .. ≤500 = medium, 500 ≤ .. ≤ 5000 nM = low binder, >5000 random binder (no specific binding).

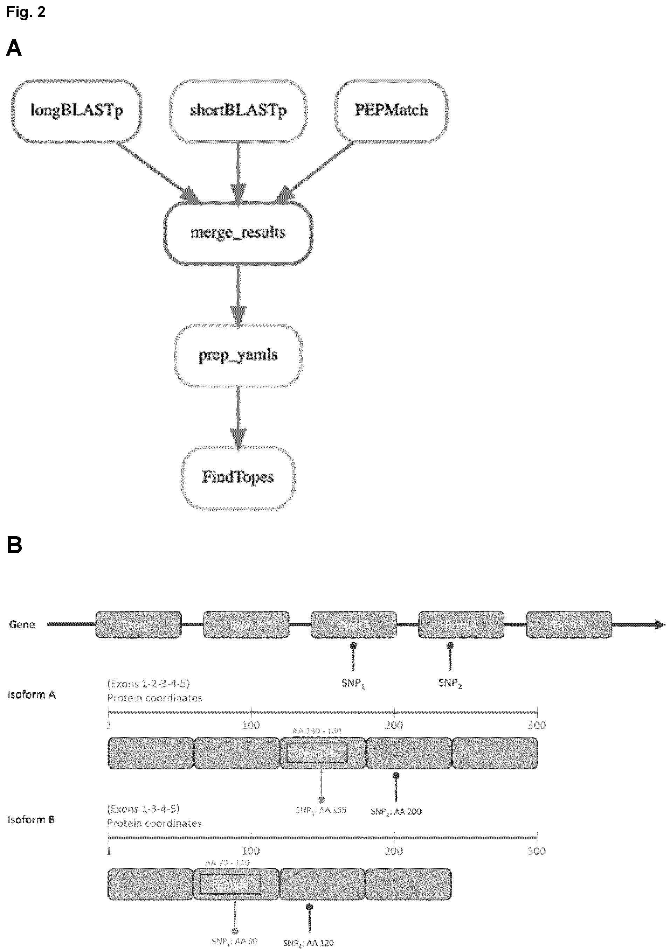

In embodiments, any SNP within the sequence of a peptide of interest may be derived or extracted from a data source, such as a (online) database. In embodiments, SNPs derived from a data source, may be annotated to their positions within a respective peptide of interest. In embodiments, after annotation of SNPs to their respective peptide of interest it may be analyzed whether the position (within the sequence of a peptide of interest) of a respective SNP is comprised within the region / site of the wildtype peptide of interest considered to represent a binding/target site of a protein of interest (e.g., the region mediating the off target binding), and/or whether it is comprised within the same gene and/or same isoform as said wildtype (wt) peptide of interest considered to be a (potential) binder of the protein of interest (see Fig. 2B). In embodiments, after determining the potential presence of a SNP within the potential binding/target region/site of a respective peptide of interest, a new (mutated) sequence of interest may be added to the list of peptides of interest, by substituting in the amino acid sequence of the respective wt peptide of interest with the amino acid representing said SNP, thereby preferably obtaining the sequence of a potential peptide of interest comprising a (known) SNP. In embodiments, also the 'edit distance' of the newly added peptides may be calculated, preferably applying a similarity and/or dissimilarity threshold, such that only SNP-comprising peptides of interest are considered for downstream analysis, which share a certain similarity to a respective wt protein of interest sequence, e.g., similar to sequence similarities disclosed herein between target peptide and peptides of interest, e.g., of > 50 %, or between 20-99,5 %. In embodiments, the resulting supplemented list of peptides of interest may be isoform specific, e.g., as both peptide and SNP locations/coordinates may depend on a specific isoform, such that their coordinates could be different despite sharing the same sequence. In embodiments, all obtained results (e.g., wt and/or SNP-comprising sequences of peptides of interest) may be reported as output, e.g., in silico and/or as a list, table, matrix or database displayed and/or saved on a storage, for further downstream analysis.

In embodiments, the identified peptides of interest, optionally also those comprising SNPs, are provided or stored comprising additional information, e.g., comprising amino acid and/or nucleic acid sequence information, information on gene-protein/peptide mapping, information on transcript-isoform mapping, information on the 'edit distance' (mutation distance) between SNP-comprising peptide of interest and wt peptide, information on wildtype/mutated annotation, information on the position of a SNP in a corresponding peptide of interest and/or information on the position of a peptide of interest and/or SNP in a corresponding (expressed) protein.

In embodiments, the edit distance reflects the distance between off-targets (peptides of interest) and target (peptides). For instance, without limitation thereto, the edit distance between WT peptide and its mutated version may be 1, when one SNP at a time is introduced. In embodiments, the 'mutated' peptide of interest (comprising one or more SNP(s)) is subsequently analysed for its sequence similarity to the at least one target peptide, wherein only SNP-comprising peptides that pass a certain, pre-determined, similarity threshold (e.g., see sequence similarity percentages herein above) are considered in subsequent analysis, and all peptides not passing the threshold are excluded from further consideration/'discarded'. In some preferred embodiments the same sequence similarity thresholds are applied as in step c. when determining the one or more peptides of interest from the (entirety of) the human proteome.

In embodiments, to determine the context of potential off-target peptides (peptides of interest) and whether they are already known or have been described before, public databases and/or in-house curated database(s) (e.g., data sources comprising mass spectrometry, and/or Co-IP, Yeast-two hybrid and/or other protein-interaction data, such as the in-house database 'PEPREP'), which preferably aggregate experimental peptide-HLA interactions and/or incorporate data from publicly available repositories, may also be considered in the present method / workflow. In embodiments this approach may provide insights on these off-target peptides and may help to assess whether to include them further in the *in silico* and/or *in vitro* downstream analysis.

In embodiments, the present method may further comprise an assessment / determining of a respective peptide of interest may potentially be presented on the surface of a cell within an HLA-complex. In embodiments, such assessment may comprise determining or predicting the affinity of a peptide of interest to a HLA allele, isoform or complex. In embodiments, information regarding such affinity a peptide of interest to a HLA molecule and/or its probability of being presented on the surface of a cell within an HLA-complex may be obtained from one or more data source, e.g., from one or more public (online) databas(es) and/or in-house (curated) database(s). In a non-limiting example, the presentation likelihood and/or presentation score and/or information regarding the affinity may be obtained using NETMHCpan or similar methods/tools. In embodiments, information regarding the presentation and/or HLA-affinity of each peptide of interest may be obtained from one or more MHC databases, such as e.g., IEDB and/or PEPREP, and/or in-house databases, preferably each comprising information on experimentally detected/verified MHC complexes.

In embodiments, finally, expression levels across healthy tissues and/or a peptide's biophysical properties are used to rank peptides of interest and evaluate the likelihood of unintended recognition/binding (off-target activity) of the protein of interest thereto.

In embodiments, a ranking score may be used for integrating the predicted affinity of (off-target) peptides of interest to the relevant HLA allele and/or their sequence similarity to the target. In embodiments, a ranking score may be predicted based on and/or using a substitution matrix.

In embodiments, all information and/or characteristics determined for peptides of interest may be integrated with each other, optionally also with information regarding the expression of a respective corresponding gene, optionally on and/or transcript level. In embodiments, in addition to the information and/or characteristics determined for peptides of interest also ranking/risk scores determined before, e.g., regarding the presentation of peptides of interest in a HLA complex, may be integrated. In embodiments, the information may be integrated in the form of a database and/or within one or more ML-models, or as other suitable data storage forms.

In embodiments, a so called 'bi-features score' may be determined, preferably to prioritize peptides of interest as 'off-targets' of a protein of interest. In embodiments, a bi-features score may be determined based on similarity to a target peptide or target amino acid motif of a protein of interest, optionally using a substitution matrix, such as e.g., BLOSUM62 substitution matrix, and/or the affinity of a respective peptide of interest to a HLA-molecule (e.g., to form a HLA-complex). which may be, for example, indicated as an IC50.

In embodiments, the 'bi-features score' is indicative of the likelihood of a respective peptide of interest being an off-target of a protein of interest (in other words, the higher the bi-features score, the higher the risk or likelihood that a respective peptide of interest is an off-target of a protein of interest). Preferably, said bi-features score is indicative of a respective peptide of interest should be considered during/for in downstream analysis.

In embodiments, the bi-features score' function (S) is defined
as $S \left(a, b\right) = \frac{f \left(a\right) + g \left(b\right)}{2}$, where: $f \left(a\right) = \left\{1, 0.66, 0.33, 0\right\} for a ∈ \left\{\left[0, 50\right), \left[50, 500\right), \left[500, 5000\right), \left[5000, ∞\right)\right\}$ $g \left(b\right) = \frac{b - {b}_{min}}{{b}_{max} - {b}_{min}} .$

In embodiments, the final peptide classification is then estimated using supervised machine learning method, k-means, e.g., with 3 clusters (e.g., 'risk categories'): ${peptide}_{rank} = {kmeans}_{3} \left(S\right) \to \left\{Low, Moderate, High\right\}$

In embodiments, a so called 'multi-features score' may be determined, preferably to prioritize peptides of interest as 'off-targets' of a protein of interest.

In embodiments, such 'multi-features score' may be considered an approach suitable for handling high-dimensional feature spaces, thereby allowing for a better integration of a peptide's (of interest) physiochemical features and other features contributing to a peptide's biological context, thereby likely also to their relevance as off-targets of a protein of interest.

In embodiments, when calculating a 'multi-features score' a feature matrix may (first) be constructed $X = \left[{x}^{1},{x}^{2},…,{x}_{n}\right] ∈ {ℝ}^{mxn}$, where *m* is the total number of peptides, *n* total number of features and *xᵢ* represents a features vector. In embodiments, such features may be selected from, without limitation thereto: affinity, BLOSUM62, stability, and/or expression etc.

In embodiments, when calculating a 'multi-features score', subsequently the features determined in a first step may be normalized, e.g., according to: $X _ norm = \left(X-μ\right) / σ or X _ norm = \left(X-X_min\right) / \left(X_max-X_min\right) .$

In embodiments, when calculating a 'multi-features score', subsequently a dimension reduction technique may be used on the, preferably normalized, data. In embodiments, a dimension reduction technique may be used, e.g., according to: $Z = DR \left(X_norm\right) ∈ {ℝ}^{mxk} where k < n and DR ∈ \left\{PCA,t-SNE,UMAP\right\} .$

In embodiments, when calculating a 'multi-features score', subsequently an unsupervised clustering may be performed. In embodiments, unsupervised clustering may be performed, e.g., according to: $\left.C=Clustering\left(Z, {k}_{clusters}\right)\right| C ∈ \left\{K-means,hierarchical,DBSCAN\right\} .$

In embodiments, when calculating a 'multi-features score', finally, a ranking may be defined as *Rank*(*peptideᵢ*) *= f*(*clusterᵢ, distance_to_centroidᵢ*)*.*

In embodiments, also non-human genomes may be considered by the present method. Preferably such step enables identifying peptides of interest, which may constitute off-targets, in pathogen and/or virus species, which are preferably hosted only by humans, e.g., HIV. In embodiments, in such consideration, of the identification of viral peptides of interest potentially being off-targets of a protein of interest, may be performed similar as in the above-described method (step(s)) used for identifying peptides of interest in the human genome. In embodiments, also the input data for viral peptides of interest may be obtained/derived (e.g., downloaded) from one or more data source, e.g., (online) databases or data resources, and may be embedded into one or more substitution matrix(es), preferably, also the same cutoffs and criteria may be applied as for the human peptides of interest (e.g., regarding length, edit distance, etc.; see above).

In embodiments, the present further comprises, preferably analogous to steps b. and c. of the method performed for the human genome/proteome, providing protein sequence information of human-hosted viruses, preferably obtained from one or more (online) data bases comprising sequence information of proteins expressed by human-hosted viruses (and/or any other human-hosted microorganism and/or pathogen), preferably comprising information on the respective amino acid sequence of said proteins, and optionally the genes encoding said proteins, and/or their nucleic acid sequence, and determining at least one viral peptide (and/or microorganism and/or pathogen peptide of interest) of interest by comparing the sequence of the at least one target peptide to the provided viral protein sequence information (and/or microorganism and/or pathogen protein sequence information), wherein the at least one viral peptide of interest (and/or microorganism and/or pathogen peptide of interest) comprises a sequence that has at least 50% sequence identity to the sequence of the least one target peptide, and optionally the same length (± 5 %, ± 15 %, ± 25 %, ± 50 %, or e.g., similar to sequence similarities disclosed herein between target peptide and peptides of interest, e.g., of > 50 %, or between 20-99.5 %, however, in embodiments the respective thresholds applied for human peptides and viral peptides (and/or microorganism and/or pathogen peptides) may be the same or may differ or vary from each other) as the least one target peptide, and wherein in step g. of the present method the data on viral peptide of interest (and/or microorganism and/or pathogen peptide of interest) is combined with the data obtained in steps. a.-f. and ranking is performed accordingly as described herein for human peptides of interest.

In embodiments, the amino acid sequence of the target peptide/motif of the protein of interest may be analyzed for potential HLA-allele binding motifs, e.g., using *in silico* binding analysis and/or prediction and/or *in vitro* binding data. In embodiments, to extend the prediction of HLA-binding motifs, the target sequence of a protein of interest may be modified/mutated one amino acid at the time. In embodiments, due to the number of different amino acids, the total number of modified target sequence versions may be L×19. In embodiments, the affinity of each modified target sequence to a HLA-molecule may then be predicted *in silico,* e.g., using NETMHCpan, as outlined above, and/or determined by *in vitro* binding assays.

In embodiments, such analysis may reveal the potential binding motif/sequence of a target peptide with a HLA-molecule/allele, which may be considered when determining how a potential HLA-complex could/would be formed, and preferably thereby enabling a prediction, which part of the sequence could be eventually exposed to the protein of interest in a subsequent *in vitro* screening.

In embodiments, input data for the present method comprises sequence and/or structural data of at least one target peptide of a protein interest, sequence and/or structural data of at least one protein of interest and/or sequence and/or structural data of at least one peptide of interest.

In embodiments, providing input data may comprise obtaining (e.g., downloading) data/information regarding the structure and/or sequence of protein(s) of interest, target peptide(s) of a protein of interest and/or peptides of interest.

In embodiments, step a., b. and/or c. comprise obtaining/providing data from one or more (online) databases, such as UNIPROT, NCBI, HPA, GTEx, Gnomad and/or Ensembl.

In embodiments, in step b. the human protein sequence information comprises (the entirety of) or at least a fraction of the (known) human proteome, or of a certain specific fraction (e.g. a certain population, certain age-range, certain ethnicity, or any other specific context (e.g., disease, organ, tissue, cell type etc.) of the human proteome.

In embodiments, in step b. the human protein sequence information is obtained, preferably at least partially, from one or more (online) databases, such as UNIPROT, NCBI, HPA, GTEx, Gnomad and/or Ensembl, and optionally further applicable sources.

In embodiments, in step b. the human protein sequence information is obtained using DBfetch or an equivalent tool or algorithm. In the context of the present invention an algorithm as DBfetch, or equivalents, may be used for obtaining protein sequence, SNP and other input data from (online) sources, e.g., databases, and may optionally also be used for updating them and/or ensuring data availability throughout the method.

In embodiments, a peptide of interest is, or comprises one or more peptides that share a certain (sequence and/or structural) similarity with a target peptide of a protein interest and may be considered a desired or undesired binder (binding*/interaction partner) of a protein of interest. In embodiments, a peptide of interest may be undesired binder of a protein of interest, such as an off-target or a peptide for which a protein of interest has an (undesired) cross-reactivity.

In embodiments, step a., b. and/or c. of the present method further comprises embedding the obtained input data into a substitution matrix.

In embodiments, input data for a peptide of interest is either i) obtained from one or more data source and/or ii) subsequently filtered, based on a comparison with a certain peptide sequence or structure, e.g., the sequence or structure of one or more target peptides, wherein only data for peptides with a certain (sequence or structural) similarity to a query peptide, e.g., target peptide sequence or structure, is i) obtained from said data source, or ii) filtered and selected for subsequent analysis. In some embodiments BLASTp, or a similar tool, is used for obtaining only peptide sequences from data resources with a certain similarity to a query peptide, e.g., a target peptide of protein of interest.

In embodiments, input data, e.g., obtained/derived (e.g., downloaded) from one or more data source, e.g., (online) databases or data resources, may be embedded into one or more substitution matrix(es).

In embodiments, input data is embedded into a BLOSUM substitution matrix, e.g., a BLOSUM62 substitution matrix. In embodiments, a PAM substitution matrix, e.g., PAM250, may be used for embedding data into a substitution matrix. In some embodiments, different embedding methods may be used, dependent on the input data and/or subsequent use thereof.

For example, by using, e.g., in certain embodiments or steps of the present method, a PAM-based method instead of, or in addition to a BLOSUM-based method, it is possible to retrieve long distanced peptides that could be missed by BLOSUM matrices, hence using PAM matrices may be advantageous in certain embodiments or method steps, as PAM is generally considered lenient and more permissive to substitutions (mismatches are less penalized) and therefore more suitable for distance homologs.

In embodiments, in addition to using one or more substitution matrix-based methods for embedding and analyzing input data, e.g., obtained from databases, additional comparisons between sequences of peptides of interest and target peptide sequences (target peptides of a protein of interest) may be employed to generate a first set of candidate peptides of interest. In embodiments, one such additional method may be a method or tool that that identifies similar peptide sequences to a target peptide sequence, e.g., using k-mer approach, such as PEPMatch.

In embodiments, after obtaining sequence and/or structural data of at least one target peptide of a protein interest, of at least one protein of interest and/or of at least one peptide of interest, further peptides (peptide sequences) of interest comprising one or more mutations over the target peptide (sequence) may be added to the input data and considered by the present method. In embodiments, such one or more mutations may comprise one or more SNPs (short nucleotide polymorphisms). In embodiments, if multiple SNPs overlap/could be comprised within the sequence of a peptide of interest, a new peptide of interest is generated for each respective SNP, such that for each SNP an additional peptide of interest is added to the sum of peptides of interest, each comprising only one SNP. In embodiments, this may also be applicable to the target itself.

In embodiments, the first list of peptides of interest obtained from the input data may be filtered or selected for further downstream analysis, based on certain criteria (cutoffs). In embodiments, the peptides of interest comprised within the input data may be filtered or, selected such as that only peptides of interest are used for further downstream analysis that: share a high, e.g., >50-60%, sequence similarity to the target peptide, and/or that have the same or similar length (e.g., of their amino acid sequence) as the target peptide.

In embodiments, the present method further comprises filtering the input data for peptides of interest sharing a high, e.g., > 50%, sequence similarity with the least one target peptide and/or having the same or similar length as the least one target peptide and/or having no gaps in the alignment.

In embodiments, once the (set of) peptides of interest that should be used for further downstream analysis is determined, each peptide of interest may be mapped to its genomic and proteomic location coordinates, e.g., based on a data source comprising sequence data and genomic and proteomic location coordinates.

In embodiments, genomic coordinates may be extracted from the one or more data sources from which input data is obtained. In embodiments, one or more data sources, e.g., online databases, may be interrogated regarding known or possible SNPs or mutations within the peptides of interest comprised within the input data.

In embodiments, said interrogation is based on or uses genomic coordinates of the peptides of interest comprised within the input data and/or obtained from one or more (online) databases, e.g., based on the sequence of the one or more peptide of interest.

In embodiments, the present method further comprises obtaining information regarding known, preferably experimentally validated, SNPs and/or mutations within the one or more peptide of interest, preferably from one or more (online) databases.

In embodiments, the obtained information on possible SNPs within the peptides of interest may be filtered, e.g., according to the population, quality and/or allele frequency (e.g., > 0.1) of said SNP.

In embodiments, the quality filtering of potential SNPs within the peptides of interest may consider only experimentally or validated reported SNPs, such that e.g., only predicted SNPs may optionally be excluded from the downstream analysis.

In embodiments a (bi-features) ranking score is used to integrate the predicted affinity of (off-target) peptides of interest, e.g., to the relevant HLA allele and/or their sequence similarity to a target peptide sequence, preferably computed using the BLOSUM (BLOcks SUbstitution Matrix) substitution matrixes, e.g., BLOSUM62.

In embodiments, leveraging supervised machine learning, a bi-feature ranking score classifies the peptides into distinct risk categories, supporting more informed prioritization of peptides regarding their potential binding to a protein of interest.

In embodiments, a bi-feature ranking score classifies the peptides into distinct risk categories scores, preferably into three categories, 'low', 'medium'/'moderate'/'intermediate', and 'high'.

In contrast, in embodiments the multivariable embedding-based ranking according to the present invention incorporates a broad range of (off-target) peptide properties, including hydrophilicity, aromaticity, gravy score and/or other physicochemical features. In embodiments, machine learning techniques (e.g., ML models, one or more Al and other suitable computational means) may be applied to classify the off-target peptides based on these embeddings, enabling risk classification without prior labeling.

Embodiments of the present invention offer the possibility to screen and identify off-targets in non-human genomes, such as viruses that are hosted by humans, which has not been described in the literature, yet.

Particularly advantageous features and effects of preferred embodiments of the present invention comprise an early SNP inclusion, MHC context integration, feature selection and ranking and/or off-targets screening in non-human genome.

In embodiments, the present method may be used to determine target peptides of a protein of interest, wherein the target peptides may comprise desired targets (interaction partners) and/or off-targets, namely peptides that are undesired interaction partners of a protein of interest. In the applications, wherein a protein of interest is a therapeutic molecule it is commonly desired to target a certain number, or only one target (e.g., target peptide), whereas other interactions may be undesired, e.g., as they are associated with, or cause side effects of the therapeutic molecule.

Hence, it may be desirable in certain embodiments to determine possible on-targets (desired targets) and off-targets of a protein of interest in a patient or patient population. In preferred embodiments determining the binding / interaction partners of a protein of interest comprises determining binding / interaction partners that may be presented in a biological context on HLA molecules as pHLA complexes. Side effects of therapeutic molecules are often caused by the off-target binding to peptides presented in pHLA complexes.

Therefore it may be advantageous to determine an optimal peptide set that may be presented in the context of pHLA complexes to a protein of interest in an *in vitro* or *in silico* interaction screening, such as an experimental *in vitro* high throughput interaction screening (HTS), to make the best prediction and/or assessment regarding potential targets and off-targets of a protein of interest.

Hence, the present invention relates in embodiments to a method for selecting a suitable, the most promising or optimal peptide-set, or pHLA set comprising a variety of different peptides for an *in vitro* or *in silico* interaction/binding screening for a protein of interest.

In embodiments more than 10, more than 25, more than 50 or more than 100 different peptides are selected for such interaction screening to a protein of interest.

In embodiments the interaction screening may be a high throughput screening (HTS).

In embodiments the present methods starts from one or more known target peptides of a protein of interest, preferably that are known to bind to the protein of interest, e.g., in the context of a pHLA complex.

In embodiments information regarding known target peptides of a protein of interest may be obtained according to the present method from one or more databases or data resources, such as online databases and/or patent databases describing target and/or off-target peptides, or binding motifs, of a protein of interest.

In embodiments the interaction screening may be performed to experimentally determine interaction and/or binding data of at least one protein (e.g., a protein of interest) with one mor more, preferably multiple, peptides, which may preferably be presented on a HLA protein/molecule forming a pHLA complex.

In embodiments the present invention relates to a method for selecting suitable, preferably the most promising or most likely, target peptides that may be presented to a protein of interest in the context of a pHLA complex, preferably in an *in vitro* or *in silico* interaction screening, preferably in an experimental interaction/binding screening regarding the binding of said peptides to the at least one protein of interest.

In embodiments, determining binding data of at least one peptide and/or at least one peptide presented within a HLA-peptide complex (pHLA) with at least one protein of interest comprises selecting / defining a pHLA set to be experimentally analyzed regarding its binding to one or more protein(s) of interest.

In embodiments, the protein of interest may be a soluble pHLA binder (pHLA-binding molecule, molecule binding to pHLA complexes) or a membrane bound pHLA binder (pHLA-binding molecule, molecule binding to pHLA complexes).

In embodiments, the protein of interest may be a soluble molecule binding to pHLA complexes (pHLA-binding molecule). In embodiments, the protein of interest may be a membrane-bound molecule binding to pHLA complexes (pHLA-binding molecule).

In embodiments, the protein of interest is a molecule capable of binding a pHLA complex. In some embodiments, the protein of interest is a genetically modified protein or antibody, or equivalent or antibody fragment.

In general, proteins of interest that are capable of binding a pHLA complex may be used in embodiments for mediating anti-tumor effects in a patient, such as mediating cell-mediated cytotoxicity (ADCC) of natural killer (NK) cells, complement-dependent cytotoxicity (CDC), antibody-dependent cellular phagocytosis (ADCP) by macrophages, or tumor cell apoptosis. Another application for proteins of interest may in embodiments be CAR-T cell therapy, or TCR-T cell therapy. Hence, the present method may be used in embodiments to develop, optimize or test therapeutic molecules of interest with regard to their binding/interaction properties to peptides or preferably pHLA complex-bound peptides of interest, e.g., in the context of developing new cancer therapeutics.

The proteins of interest that are capable of binding a pHLA complex may be used in embodiments for optimizing or developing research applications, e.g., when a protein of interest is conjugated to a fluorescent agent to detect the expression of target peptides or pHLA complexes on tumor cells.

In some embodiments, the molecule of interest being an equivalent of a receptor, an antibody or antibody fragment thereof is any biomolecule capable of recognizing and/or specifically binding antigens and/or capable of (specifically) binding to pHLA complexes. In embodiments, the term 'any biomolecule capable of recognizing and/or specifically binding antigens and/or capable of (specifically) binding to pHLA complexes' also comprises DARPins.

In embodiments, a marker characterizing such a pHLA set of interest is the mutation distance to the target peptide sequence. Commonly, a distance of 1 means that the peptide (here preferably the peptide presented within a HLA-peptide) differs by 1 mutation (including insertions and deletions) from the target. Commonly, the maximum number of mutations is defined by the target length. In embodiments, it is preferred to include all possible peptides with a distance of 1 and an equal mix of all other mutations.

In embodiments the final output list / sum of peptides of interest that may be used for an in vitro interaction screening may further be complemented or comprises sequence variation of the at least one target peptide. In embodiments, a further method step comprises systematically substituting (*in silico*) in the sequence of the target peptide, provided in a., each amino acid (one at a time), optionally by 19 different amino acids respectively and consecutively (herein also termed 'Xscan'), preferably to determine HLA binding strength or specificity (e.g., using NetMHCpan affinity and optionally other *in silico* prediction tools, to determine e.g., the IC50) for each amino acid sequence position-of the *in silico* modified target peptide sequence, thereby identifying critical residues for peptide-MHC complex formation and target protein binding. In embodiments the sequence variation of the target peptide with the best/binding properties to a HLA molecule are also added to the sum of peptides of interest for subsequent *in silico* and/or *in vitro* analysis

In embodiments, the method further comprises supplementing / complementing the list of the peptide(s) of interest generated in step g. by a list of mutated target peptide sequences, wherein each mutated target peptide sequence comprises one amino acid substitution over the wild type sequence of the at least one target peptide, preferably wherein each consecutive amino acid of the entire sequence length of the at least one reference peptide has been mutated accordingly, and wherein preferably each mutated target peptide sequence is provided with a binding affinity (e.g., as IC50) to a HLA molecule, e.g., predicted according to step f. In other words, in embodiments the present method further comprises a step of determining a list of mutated target peptide sequences by ('step wise') replacing each amino acid of the entire target peptide sequence by a different amino acid to generate a list of mutated target peptide sequences each comprising one mutation over the wt target peptide sequence, respectively. In embodiments, also a binding affinity to a HLA-molecule is determined, as described herein, e.g., in step e., for each mutated target gene sequence, which preferably is then also considered when selecting peptides of interest for subsequent interaction screenings.

In embodiments, the present method comprises multiple individual workflows, wherein each is preferably designed to perform specific tasks.

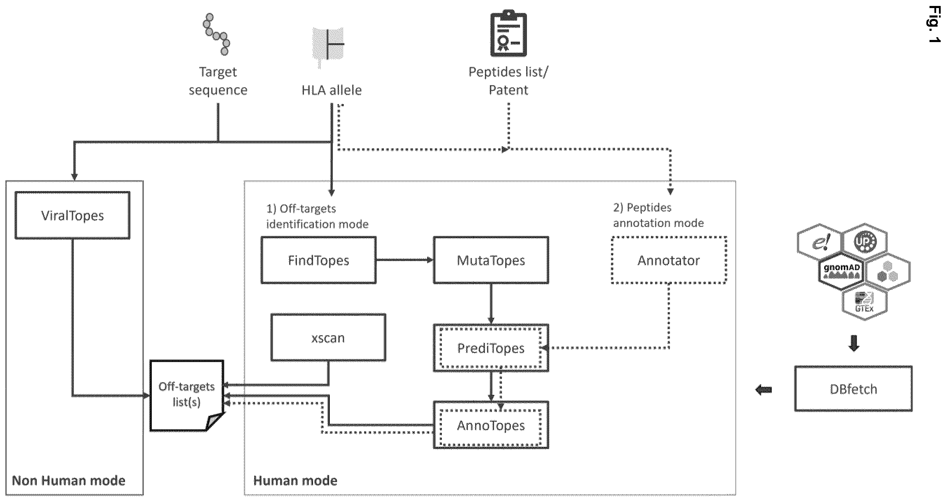

In embodiments, the present method may be performed in two different modes, the 'off-target identification' mode and the 'peptides annotation' mode (see, e.g., Fig. 1). In embodiments, said modes may comprise different method steps, varying depending on the mode.

In one specific embodiment the present method may be performed in the 'off-target identification' mode and comprise one or more of the following input parameters: HLA allotype specifications (standard nomenclature), target sequence of any length (typically 8-15 amino acid length), mismatch threshold, population and/or allele frequency threshold.

In some embodiments, the workflow of the 'off-target identification' mode comprises on or more of the following method steps, preferably according to the embodiments outlined herein, comprising:
- identifying peptides of interest ('off-targets') in the human genome, namely peptides with a sequence similarity to the target peptide of a protein of interest, preferably comprising a sequence similarity comparison step and employing a substitution matrix for data embedding.
- filtering the input data for peptides of interest sharing a high, e.g., > 50%, sequence similarity with the least one target peptide and/or having the same or similar length as the least one target peptide.
- obtaining information regarding known, preferably experimentally validated, SNPs and/or mutations within the one or more peptide of interest, e.g., preferably from one or more (online) databases, and subsequently filtered, e.g., according to the population, quality and/or allele frequency (e.g., > 0.1) of each respective SNP, wherein optionally quality filtering of potential SNPs may consider only experimentally or validated reported SNPs.
- determining the probability of a peptide of interest being presented on a cell and/or the affinity of a respective peptide of interest to a HLA-allele of interest, optionally also comprising *in silico* measurements of interaction strength or affinity between a respective peptide of interest and a HLA-molecule.
- obtaining and considering information regarding the presentation and/or HLA-affinity of each peptide of interest may be also, preferably comprising information on experimentally detected/verified MHC complexes.
- aggregate and one ore more annotations by determining a 'bi-features score' and/or a 'multi-features score', preferably to rank peptides of interest regarding the risk of them being likely off-targets of a protein of interest.
- providing an output, preferably comprising ranked and fully annotated peptides of interest (e.g., further comprising binding predictions with experimental validation status and gene expression).
- repeating one or more of the above steps to determine peptides of interest within non-human genomes in pathogen and/or virus species, which are preferably only hosted by humans, e.g., HIV.
- analyze the amino acid sequence of the target peptide/motif of the protein of interest for potential HLA-allele binding motifs, e.g., using in silico binding analysis and/or in vitro binding data.

In some embodiments, the output data provided by embodiments of the above method comprise on or more of the following, preferably according to the embodiments outlined herein, comprising:
- information on sequences of peptides of interest (potential off-targets) with full annotations.
- information regarding predicted binding affinities of peptides of interest with HLA-molecules (IC50 values).
- information on the likelihood of a peptide of interest being presented von a cell, e.g., comprising a score and/or percentile.
- a classification categories (high/medium/low), e.g. bi-/multi-feature scores.
- information regarding existing/known experimental validation of peptide properties, e.g., HLA- binding and/or interaction partners.
- information regarding tissue-specific context data.
- information regarding gene expression of a corresponding peptide of interest.
- information on synthetic peptides.

In one specific embodiment the present method may be performed in the 'peptides annotation' mode and comprise one or more of the following input parameters: HLA allotype specifications (standard nomenclature), target sequence of any length (typically 8-15 amino acid length) and/or peptide list.

In some embodiments, the workflow of the 'peptides annotation' mode comprises on or more of the following method steps, preferably according to the embodiments outlined herein, comprising:
- execute an annotation to annotate peptides to a gene and/or protein.
- predict *in-silico* measurements of peptide binding comprising predicting affinity between peptides and MHC / HLA molecules.
- add experimental annotation data according to MHC databases (e.g., IEDB + PEPREP (in-house MHC resource)) .
- aggregate obtained data and add more annotations, then determined a risk-ranking of the peptides of interest regarding their likelihood of being off-targets.
- output ranked and fully annotated peptides of interest (potential off-targets), preferably comprising information on binding predictions, optionally with experimental validation status, and/or gene expression.

In some embodiments, the output data provided by embodiments of the above method comprise on or more of the following, preferably according to the embodiments outlined herein, comprising:
- information on sequences of peptides of interest (potential off-targets) with full annotations.
- information regarding predicted binding affinities of peptides of interest with HLA-molecules (IC50 values).
- information on the likelihood of a peptide of interest being presented von a cell, e.g., comprising a score and/or percentile.
- a classification categories (high/medium/low), e.g. bi-/multi-feature scores.
- information regarding existing/known experimental validation of peptide properties, e.g., HLA- binding and/or interaction partners.
- information regarding tissue-specific context data.
- information regarding gene expression of a corresponding peptide of interest.
- information on synthetic peptides.

In embodiments, step b. comprises one or more of the following input parameters: database identifiers and API endpoints, authentication credentials and access protocols and/or database-specific filtering thresholds. update frequency parameters (monthly/yearly), data quality validation criteria, version control and/or cross-reference mapping capability.

In embodiments, in step b. database-specific filtering thresholds comprise SNP database (exon regions only), tools specific input, expression database (tissues with available samples-meta info), and/or protein database. In embodiments, data from protein databases comprise: experimentally validated protein sequences (flag = sp (swiss-prot)), isoforms mapping availability and/or viruses and bacteria protein sequences that hosted by human.

In embodiments, step b. comprises one or more of the following features and steps:
1. database query engine, preferably comprising one or more of i) execute parallel api calls to configured databases, implement retry logic and error handling, and/or iii) apply database-specific filters; and/or
2. data standardization pipeline, preferably comprising one or more of: i) convert heterogeneous formats in unified schemas when possible, ii) unify identifiers across database sources and/or iii) resolve cross-reference mapping and other conflicts; and/or
3. quality control processor, preferably comprising one or more of: i) validate data integrity and completeness, ii) highlight inconsistencies, missing annotation and excluded data, and/or iii) generate quality metrics and processing summaries; and/or
4. integration and indexing engine.

In embodiments, step b. comprises one or more of the following output data structures:
1. protein sequence repository, preferably comprising one or more of i) FASTA filtered sequences, ii) protein and isoforms sequences and/or iii) cross-reference mapping (Uniprot, Ensembl, etc.), and/or
2. population genetics dataset, preferably comprising one or more of i) indexed SNP variant files (vcf format), ii) population frequencies, iii) quality metrics, and/or d. SNP annotations files (genomic coordinates); and/or
3. gene expression matrix, preferably comprising one or more of i) tissue-specific expression levels (tpm/ntpm/ptpm), cell-level and/or ii) gene and transcript level; and/or
4. cross-reference mapping tables, preferably comprising one or more of i) gene id to protein id mappings, ii) transcript id to isoform id mappings, iii) genomic coordinates to gene positions, d. gene type annotation source and/or e. version tracking.

In embodiments, step c. comprises one or more of the following steps:
1. BLASTp dual-mode search, preferably comprising i) short mode: BLOSUM62 matrix, e-value 1e⁻⁶, and/or ii)long mode: PAM250 matrix, e-value 1e⁻⁶; and/or
2. PEPMATCH k-mer search, preferably comprising i) deterministic k-mer decomposition algorithm, and/or ii) distance-based similarity scoring; and/or
3. result integration process, preferably comprising i) duplicates removal algorithm, and/or cross-validation algorithm.

In embodiments, step c. comprises one or more of the following processing steps: target peptide preprocessing, preferably comprising validate input sequence format and protein info and/or generate search parameters for each algorithm; dual BLASTp search, preferably comprising short: execute BLOSUM62-based search, preferably comprising conservative substitution scoring matrix, detect highly similar peptides that could be missed by PEPMATCH and/or statistical significance threshold: e-value ≤ 1e-6; and/or long: execute PAM250-based search, preferably comprising lenient substitution matrix for distant homologs, enhanced sensitivity for divergent sequences and/or extended evolutionary distance detection; PEPMATCH k-mer analysis, preferably comprising decompose target into overlapping k-mers (k=2), execute deterministic similarity matching and/or identify divergent peptides missed by BLASTp algorithms; and/or result integration and deduplication, preferably comprising merge results from all search algorithms, remove duplicate identifications across methods, and/or generate unified candidate off-target list; multi-criteria filtering, preferably comprising sequence dissimilarity constraints (50-60% maximum dissimilarity), exact length matching, relaxed length, and/or exclude gap-containing peptides; and/or genomic coordinate mapping, preferably comprising map peptide sequences to protein database entries, extract genomic coordinates and chromosomal positions using cross-reference tables and/or generate cross-reference annotations.

In embodiments, step c. comprises one or more of the following input parameters: target peptide sequence (typically anything between 8-15, or 5-20 amino acids), reference protein databases (Uniprot), configurable similarity thresholds (default: 50-60% dissimilarity, or even less (see also embodiments above)), and/or gap penalty configurations (default: gaps disabled).

In embodiments, step c. comprises one or more of the following algorithm-specific settings: BLASTp short mode, preferably comprising substitution matrix: BLOSUM62, word size: 3, and/or gap penalties: disabled; and/or BLASTp long mode, preferably comprising substitution matrix: PAM250, and/or word size: 2; and/or PEPMATCH configuration, preferably comprising k-mer size: configurable (default: 2) and/or distance metric: hamming.

In embodiments, step c. comprises one or more of the following output data structure: comprehensive off-target dataset, preferably comprising peptide sequences: amino acid sequences, and/or edit distance scores; and/or source protein information, preferably comprising protein identifiers and annotations; and/or genomic coordinates, preferably comprising chromosomal positions (grch38/hg38), gene symbol mappings, and/or transcript/isoform variant information; and/or algorithm provenance, preferably comprising detection method flags (blast-short/long, PEPMATCH), and/or cross-validation status.

In embodiments, step c. comprises one or more of the following specific technical features: dual-matrix BLASTp implementation, preferably comprising parallel execution of BLOSUM62 and PAM250 substitution matrices, and/or captures both close evolutionary relationships (BLOSUM62) and distant homologs (PAM250), and/or addresses coverage limitations of single-matrix approaches; dual-algorithm integration for off-target discovery, preferably comprising systematic combination of BLASTp and PEPMATCH for comprehensive off-target identification, complementary detection capabilities, and/or cross-algorithm validation ensuring comprehensive peptide coverage; complementary coverage optimization, preferably comprising based on empirical analysis of detection patterns and/or systematic approach leverages probabilistic and deterministic method.

In embodiments, step d. comprises one or more of the following features or steps for integrating SNPs by generating peptide of interest variants through systematic integration of single nucleotide polymorphism that are preferably associated with selected population.

In embodiments, step d. and/or e. comprises one or more of the following steps:
1. Genomic-based coordinate SNP extraction, preferably comprising i) query population-specific SNP databases, ii) extract SNPs using off-target genomic coordinates and/or iii) apply multi-criteria filtering pipeline; and/or
2. variant effect prediction and annotation, preferably comprising i) execute VEP (variant effect predictor) annotation pipeline, ii) determine and extract proteomic coordinate mappings, iii) determine alternative alleles, d. calculate variant impact scores (preferably three categories, 'low', 'medium'/'moderate'/'intermediate', and 'high') and functional consequences and/or e. generate transcript-specific variant annotations; and/or
3. isoform-specific coordinate, preferably comprising i) cross-reference SNP positions with off-target peptide coordinates, ii) validate gene identity and isoform compatibility, iii) ensure isoform-level coordinate consistency, and/or d.filter for overlapping genomic positions;
4. generate off-target peptide variant, preferably comprising i) systematic amino acid substitution at SNP positions, and/or ii) apply substitution logic across all validated SNP -peptide pairs;
5. sequence similarity validation, preferably comprising i) calculate edit distance for newly generated mutant peptides, ii) apply dissimilarity rate thresholds (consistent with step c. above), iii) exclude peptides with high dissimilarity (> threshold), and/or d. maintain sequence diversity (remove duplicates due to mutations);
6. comprehensive dataset assembly, preferably comprising i) integrate wildtype and mutant peptide variants, ii) generate isoform-specific result sets and/or iii) maintain complete tracking for variant origin (isoform, allele, position and impact).

In embodiments systematic amino acid substitution at SNP positions comprises extracting wildtype (wt) amino acid at SNP coordinate, substituting with SNP-derived amino acid variant (alternative allele), generating mutated off-target peptide sequence and/or maintaining peptide length consistency.

In embodiments a multi-criteria filtering pipeline according to step d. comprises quality validation filter (experimentally validated ANPs only), allele frequency threshold (≥ 0.1 population frequency), coding SNPs, an/or exclude computationally predicted variants without experimental evidence.

In some embodiments, only peptides of interests for which transcript annotation information is available (e.g., obtained in step b., c. and/or d.) will be subjected to SNP analysis in step d.; if no transcript annotation of the protein, in which the peptide of interest is predicted to be comprised, is available/could be obtained, the peptide (wt) will not be analysed in step d. regarding potential SNPs, but will, however, preferably remain in the overall sum/pool of peptides of interest for further consideration and subsequent analysis.

In embodiments, step d. comprises one or more of the following input parameters: off-target genomic coordinates from step c., population identifiers (gnomad project), quality control thresholds, preferably comprising allele frequency minimum: ≥ 0.1, quality validation: experimental evidence required, VEP configuration parameters and/or sequence similarity constraints (inherited from step c.); SNP database integration, preferably comprising primary sources: gnomad, population stratification categories, quality score thresholds (FILTER = "PASS"), functional annotation requirements; and/or isoform processing, preferably comprising transcript annotation (Ensembl), coordinate system: grch38/hg38 genomic coordinates , and/or cross-reference validation: gene symbols, gene and transcript IDs, Uniprot & isoforms.

In embodiments, step d. comprises one or more of the following output data structure: peptide variant information, preferably comprising wildtype peptide sequences with genomic coordinates and transcript id, SNP -derived mutant off-target sequences and/or updated edit distance scores; genomic annotation data, preferably comprising gene identifiers and protein mappings, transcript and isoform specifications, and/or chromosomal coordinates; VEP functional impact predictions, population genetics metadata, preferably comprising SNP identifiers (rs numbers) and allele frequencies, and/or quality validation status and evidence sources; provenance tracking, , preferably comprising wildtype vs. mutant classification flags, SNP -to-peptide mapping relationships and/or isoform-specific coordinate systems.

In embodiments, special technical features step d. comprises one or more of the following population-stratified off-target variant generation, e.g., first systematic integration of population genetics with off-target prediction, automated SNP-to-peptide coordinate mapping across isoforms, and/or population-specific risk assessment for drug off-target effects, genomic coordinate-based SNP integration, e.g., direct coordinate mapping from off-target identification to variant databases, isoform-specific coordinate validation and cross-referencing and/or systematic quality filtering for experimental validation; comprehensive variant effect integration, e.g., VEP-based functional annotation of off-target variants, multi-level coordinate validation (genomic → transcript → protein-isoform) and/or edit distance preservation across wildtype and mutant peptide sets; population-specific drug safety framework, e.g., integration of allele frequency data with off-target predictions, population stratification for personalized risk assessment, and/or systematic expansion of off-target space through genetic variation.

In embodiments, step e. comprises one or more of the following steps or features for integrating *in-silico* predictions (binding and presentation) and may be considered a validation step to distinguish high-priority off-targets from low-risk candidates through systematic integration of predictive algorithms preferably with experimental evidence databases, e.g., IEDB and the in-house curated database PEPREP.

In embodiments, step e. comprises one or more of the following input parameters: HLA allotype specifications (standard nomenclature), peptide sequences (8-15 amino acid length), and/or peptide's protein-id.

In embodiments, step e. comprises one or more of the following steps:
1. HLA prediction, preferably comprising a. execute NetMHCpan predictions with the selected HLA allotype, b. generate in-silico prediction profile, and/or c. classify off-targets by binding, presentation strength, and/or
2. experimental evidence cross-validation, preferably comprising
   a. query IEDB (immune epitope database) for experimental data, preferably comprising i. search for identical peptide sequence, ii. retrieve experimental information (protein, HLA binding info, tissue, diseases), iii. validate prediction against experimental, and/or iv. repeat on protein level;
   b. query PEPREP for additional validation, preferably comprising i. access internal experimental data, ii. retrieve all relative experimental information and/or iii. obtain binding information; cross-reference between the two resources; and/or
3. Contextual risk stratification, preferably comprising
   a. integrate prediction and experimental evidence, preferably comprising i. apply weighting metric to prediction based on experimental info, ii. mark off-targets with confirmed experimental info, iii. mark the discrepancies between predictions and experiments, iv. generate confidence scores for the prediction;
   b. apply biological context analysis, preferably comprising i. assess tissue/disease relevance, ii. evaluate clinical information if available, and/or iii. generate first risk classification,
4. urgent off-target identification approach; preferably comprising a. generate actionable risk categories, preferably comprising i. urgent: high binding + high presented + experimental evidence, ii. important: strong prediction (binding + presentation) + partial experimental evidence (e.g., different HLA allotype), iii. study/assess: moderate predictions + limited experimental support (e.g., only protein was found), and/or iv. low priority: weak predictions + no experimental confirmation.

In embodiments, step e. comprises one or more of the following output data structure: binding affinity predictions (IC50 values), presentation score and percentile, classification categories (strong/weak/non-binder), experimental validation flags, and/or tissue-specific context data.

In embodiments, step e. comprises one or more of the following specific technical features: 1. prediction-validation integration intelligence, 2. contextual risk intelligence system, e.g., a. multi-database context evaluation, b. experimental evidence, and/or c. experimental evidence weighting, 3. urgent off-target classification framework, and/or 4. dual-database validation architecture, e.g., a. public database integration for broad and community-proven experimental coverage, b. proprietary database integration (PEPREP) for specialized validation, c. enhance confidence through cross-database validation, and/or d. comprehensive experimental evidence aggregation and analysis.

In embodiments, the step g. comprises a bi- and/or multi-dimensional scoring, preferably configured to integrate biological annotations, gene expression, and physiochemical properties for comprehensive off-target risk stratification. In embodiments step g. comprises a comprehensive annotation/integration of one or more of: multi-source biological annotations, the urgent score from 4, gene expression data (gene-level and transcript-level), tissue-specific expression profiles and/or (preferably cross-referenced), preferably providing a unified annotation of datasets for downstream scoring.

In embodiments, step g. comprises one or more of the steps/features selected from: (i) calculating sequence-based: gravy scores, molecular weight and amino acid composition, isoelectric point and charge distribution, secondary structure propensities, and/or aromatic content and flexibility measures, and/or (ii) estimating features correlation matrices, and/or (iii) applying correlation analysis to identify informative features and/or (iv) filtering redundant or non-informative physicochemical features.

In embodiments, step g. comprises a bi-Features risk scoring algorithm comprising: calculating BLOSUM62-based sequence similarity scores, integrating HLA binding affinity data (IC50 values), applying feature normalization, applying dual-parameter scoring function for risk assessment, executing supervised clustering for clustering and/or generating preliminary risk classifications.

In embodiments, step g. comprises a multi-Features advanced scoring comprising: (i) constructing high-dimensional feature matrices, preferably comprising filtering physicochemical properties, and/or sequence based metrics, and/or HLA prediction metrics and/or gene expression data, and/or (ii) applying feature normalization and dimensionality reduction, and/or (iii) executing unsupervised clustering for clustering and/or (iv) generating risk classifications.

In embodiments, step g. comprises a machine learning-based classification comprising: implementing supervised k-means clustering (default: 3-cluster) and/or implementing unsupervised methods (dimension reduction: PCA, t-SNE, UMAP; clustering: kmeans, hierarchal, DBSCAN) and/or generating risk categories based on multi-dimensional analysis and/or applying ranking algorithms for final prioritization.

In embodiments, the step of identifying potential, cross-reactive, off-target peptides of interest within human-hosted microorganism proteomes comprises one or more of the following steps:
1. human-hosted pathogen database curation, preferably comprising query pathogen taxonomy databases for human-associated species, and/or filter organism databases by host specificity criteria, preferably comprising viruses (human-tropic strains and variants), bacteria (human microbiome and pathogenic species), parasites (human-infective lifecycle stages), fungi (human-associated opportunistic pathogens), maintain updated pathogen proteome repositories, and/or maintain version information; and/or
2. cross-species similarity search, preferably comprising execute identical algorithm framework as in step c., preferably comprising BLASTp dual-mode implementation (BLOSUM62 + PAM250), PEPMatch k-mer analysis for deterministic matching, and/or parallel algorithm execution with result integration; apply consistent filtering criteria, preferably comprising length matching requirements (exact target length), edit distance constraints (default 50-60% dissimilarity), and/or gap elimination (no gap-containing alignments); and/or
3. pathogen-specific result processing, preferably comprising cross-reference identified peptides with pathogen annotations, validate organism classification and human-host relationships and/or maintain organism provenance and taxonomic information; and/or
4. human cross-reactivity assessment, preferably comprising evaluate potential for molecular mimicry with human targets, assess immunological cross-reactivity risks, and/or generate pathogen-specific risk classifications.

In embodiments, the step of identifying potential, cross-reactive, off-target peptides of interest within human-hosted microorganism proteomes comprises one or more of the following input parameters: target peptide sequences from primary analysis, human-hosted pathogen proteome databases, and/or organism filtering criteria, preferably comprising host specificity requirements (human-associated only) and/or pathogen classification categories (virus, bacteria, parasite, fungi); and/or similarity search parameters (preferably inherited from step c.).

In embodiments, the step of identifying potential, cross-reactive, off-target peptides of interest within human-hosted microorganism proteomes comprises one or more of the following output data structures cross-species off-target list, preferably comprising pathogen off-target matches (preferably comprising sequence with edit distance scores, organism source identification and taxonomic classification, and/or pathogen-specific annotations and functional categories if available), organism metadata, preferably comprising pathogen taxonomy and classification information, strain and variant information, and/or database provenance and version tracking.

In some embodiments, one or more of the following preferred four criteria for choosing the pHLA set to be experimentally analyzed may be applied:
1. The pHLA set may preferably be as diverse as possible, across all numbers of mutations and all amino acids. This may ensure that a pHLA set carries as little bias as possible.
2. The minimal number of peptides in the pHLA set may preferably be at least at about 300 peptides.
3. Preferably, the chosen peptides are of physiological relevance. In embodiments, peptides of physiological relevance are commonly found in the human peptidome.
4. Preferably, a pHLA set may be composed of molecules binding and not-binding to an actual therapeutic molecule. However, in some embodiments, a pHLA set may be enlarged or redefined after the first round of wet lab experiments, as this may be difficult to determine in advance.

In embodiments, a pHLA set may be composed of ~ 50% non-binder and ~ 50% binder to an actual therapeutic molecule.

In embodiments, the pHLA set that is experimentally analyzed regarding its binding to one or more protein(s) of interest in an *in vitro* screening according to the present invention, is selected / defined by a method comprising computational means, e.g., comprising one or more machine learning approach(es) or models and/or other applicable computational means and approaches for selecting suitable reference peptide to be presented in pHLA complexes.

In embodiments, the selection approach of suitable peptides, e.g., to be presented in the context of pHLA complex to the protein of interest, in the *in vitro* screening according to the present invention comprises in one or a first step the selection of a single target peptide sequence for a protein of interest.

In embodiments, said step further comprises selecting information/data regarding the HLA type, which then serves as the input to a proprietary bioinformatics pipeline according to the present invention for selecting the pHLA set to be tested *in vitro.*

In embodiments, said pipeline generates a dataset of predicted peptides or pHLA complexes by assessing sequence similarity between peptides of interest, biophysical properties of said peptides in interest, and/or functional tissue-specific presentation likelihoods of said peptides in interest, e.g., in the context of a pHLA complex.

In embodiments, to enhance prediction confidence of the approach/pipeline for selecting a pHLA set that is to be experimentally analyzed, supporting annotations are added from a database (e.g., an online and/or in-house curated database (such as PEPREP)), which aggregates experimental peptide-HLA interactions and/or incorporates data from publicly available repositories (databases).

In embodiments, the pipeline then predicts pHLA candidates that may be included into the set of peptides or pHLA complexes to be tested for a binding to the protein of interest, e.g., *in vitro* or *in silico.* Such candidates preferably represent and/or comprise potential off-targets for a protein of interest (e.g., an antibody, fragment thereof or DARPIN).

In embodiments, the predicted peptides or pHLA complexes are computationally ranked according to the present method, preferably according to multiple criteria. In embodiments, said criteria used for ranking the predicted peptides or pHLA candidates, comprise biochemical and/or structural characteristics. In embodiments, based on this ranking, an 'informed' subset of peptides is selected for further experimental analysis in the *in vitro* screening according to the present invention.

In embodiments, the experimental determination of the binding data of the at least one peptide and/or the at least one peptide presented within a HLA-peptide complex (pHLA) with at least one protein of interest comprises:
a. performing high throughput binding experiments / analysis / screening of at least one protein of interest to the set of peptides or pHLA complexes selected previously.

In embodiments, the invention relates to a computer-implemented method for predicting one or more peptide (sequences) to be analysed in an interaction / binding screening regarding their binding to at least one protein of interest comprising one or more of the following steps:
- providing sequence and/or structural data of at least one peptide of interest,
- providing sequence and/or structural data of at least one target peptide or target motif of the at least one protein of interest
- providing sequence and/or structural data of at least one protein of interest,
- providing binding / interaction data on at least one protein of interest to target peptides or information regarding target peptides of the at least one protein of interest,
- employing a (trained) machine learning (ML)-based model or artificial intelligence (AI), to predict one or more possible peptides, preferably at least their amino acid sequence, for a binding or interaction screening to the at least one protein of interest to obtain (experimental) binding data of the at least one protein of interest to different (reference) peptides.

In embodiments, the at least one reference peptide is to be presented in the binding or interaction screening within a HLA-peptide (pHLA) complex.

In embodiments, the (experimental) determination of the binding data in an binding or interaction screening comprises the following steps:
i. providing at least one peptide of interest predicted in step g. (likely) binding to the protein of interest,
ii. supplying HLA-proteins, preferably biotinylated HLA-proteins, to the at least one peptide of interest and allowing the HLA -proteins to bind to the at least one peptide of interest, thereby forming pHLA complexes,
iii. immobilizing the formed pHLA complexes on a solid support, preferably on a streptavidin-coated solid support,
iv. contacting the immobilized pHLA complexes with at least one protein of interest,
v. determining binding data of the individual pHLA complexes with the at least one protein of interest.

In embodiments, the binding or interaction data (or properties) determined in the interaction screening comprise the binding strength, preferably comprising the dissociation constant (KD), of the at least one protein of interest to at least one reference peptide.

In embodiments, wherein the at least one peptide comprises at least one mutation with respect to a known (optimal) target peptide motif of the at least one protein of interest.

In embodiments, the interaction screening is performed experimentally and/or *in silico,* e.g., by one or more algorithm, machine learning (ML)-based model or artificial intelligence (Al).

In embodiments, the data obtained from the interaction screening is subsequently used to determine and/or compare potential off target-binding of the at least one protein of interest.

In embodiments, the data obtained from the interaction screening is subsequently used to determine and/or compare potential peptides of interest constituting a off-target binding of the protein of interest.

In embodiments, sequence and/or structural data comprises information on the amino acid sequence, the three-dimensional structure, the two-dimensional structure, the SMILES string, the InChl, the InChlKey, the chemical structure, any other biochemical property of a biomolecule, e.g. such as electrostatic patches, and/or other molecule features, optionally wherein any of the afore were predicted by a ML model or AI.

In embodiments, the pHLA complex is present in the human immunopeptidome or a synthetic pHLA complex.

In embodiments, the protein of interest is a TCR-like antibody (TCRm) or fragment thereof.

In embodiments, the protein of interest is a T cell receptor or fragment or equivalent thereof.

In embodiments, the protein of interest is a receptor, antibody, antibody fragment or equivalent thereof, wherein the equivalent of a receptor, an antibody or antibody fragment thereof is any biomolecule capable of recognizing and/or specifically binding antigens and/or capable of (specifically) binding to pHLA complexes, such as, e.g., DARPINs.

### DETAILED DESCRIPTION

The present invention generally relates to computer-implemented methods for predicting or selecting (suitable or optimal) (reference) peptides, which may preferably be presented in the context of a pHLA complex to at least one protein of interest, e.g., a receptor, antibody, antibody fragment or equivalent of, receptor, immunoglobulin or DARPin, wherein the (reference) peptides that may preferably be presented in the context of a pHLA complex are analysed in an *in vitro* or *in silico* interaction screening, preferably in an experimental interaction/binding screening regarding their binding to the at least one protein of interest.

Herein, the input data for the computational model(s), e.g., ML-models or AI, used in the context of the present method preferably comprises sequence and/or structural data and/ore interaction/binding data.

In the context of the present application, 'sequence data' comprises any biological sequence data, e.g., amino acid sequence data and/or nucleic acid sequence data. Hence, in embodiments sequence data comprises data on the amnio acid sequence of a peptide or protein and/or nucleic acid sequence data of nucleic acids encoding a peptide or protein referred to herein. In embodiments sequence data comprises data on the amnio acid sequence of a peptide or protein of interest. In embodiments sequence data comprises nucleic acid sequence data of nucleic acids encoding a peptide or protein of interest.

In the context of the present invention, 'structural data' comprises any biological data on the structure, e.g., the 2- and/or 3-dimensional structure, of a protein or peptide.

In the context of the present invention, the sequence and/or structural data may comprise information on the amino acid sequence, the nucleic acid sequence encoding a respective peptide or protein, the three-dimensional (3D) structure, the two-dimensional (2D) structure, the SMILES string, the InChl, the InChlKey, the chemical structure, any other biochemical property of a biomolecule, and/or other molecule features.

In the context of the present invention, 'interaction data' or 'binding data' preferably comprises the experimentally determined interaction and/or binding data of at least one protein (e.g., a protein of interest) with another protein, peptide or preferably a peptide presented on a HLA protein/molecule forming a pHLA complex.

In general, an off-target binder of a protein of interest, e.g., an antibody or receptor, is preferably a protein or peptide of interest to which a protein of interest binds besides its target peptide (motif) or target protein, which may constitute an undesired interaction partner as it, e.g., diminishes or impairs specific binding to a target protein (specificity) and/or as it may cause or is suspected of causing, side effects, e.g., in a patient receiving the protein of interest as a therapeutic molecule or medicine.

The 'interaction data' or 'binding data' or 'data on the interaction / binding properties' provided herein, that is preferably provided as an input to the computational model(s), preferably comprises experimentally determined data on the interaction and/or binding or interaction and/or binding properties of at least one protein to at least one peptide. The interaction or binding data comprises in preferred embodiments at least data indicating the binding strength, data regarding the positive or negative detection of binding/interaction, data on the duration of the binding, the association rate constant (kass), the dissociation rate constant (kdiss) and/or the dissociation constant (KD). In embodiments, the training of the AI or ML-model on positive and negative binding data is particularly advantageous to ensure that detected negative interactions (no binding) are true negatives and not assay artefacts.

The 'interaction data' or 'binding data' or 'data on the interaction / binding properties' predicted herein, e.g., in method step e. according to the invention, preferably comprises data regarding the binding or interaction properties of the at least one peptide of interest, preferably when comprised within a pHLA complex, to the at least one protein of interest. In embodiments, the predicted data preferably comprises data indicating the binding strength, data regarding the positive or negative detection of binding/interaction, data on the duration of the binding, an estimated association rate constant (kass), an estimated dissociation rate constant (kdiss) and/or an estimated dissociation constant (KD). In embodiments, the 'interaction data' or 'binding data' or 'data on the interaction / binding properties' may further comprise one or more potential target motifs of a protein of interest, optionally also more than one proposed target motif, e.g., ranked according to expected binding properties to the respective protein of interest. In embodiments, binding or interaction properties comprise the binding strength, preferably comprising the dissociation constant (KD), and/or the probability or likelihood of binding of the at least one protein of interest to the at least one peptide of interest, preferably when presented within a pHLA complex. In embodiments the likelihood or probability predicted by the computational model(s), e.g., in step e., comprises a score or value indicative of the likelihood and/or strength of the predicted binding between a protein of interest to a peptide of interest, preferably when comprised or presented in a pHLA complex, e.g., a value between 1 (binding) and 0 (no binding), e.g., with a threshold of 0.5 between the two classes, or another threshold, e.g. if more than 2 classes are used.

In embodiments, a HLA binding threshold, e.g., employed in method step f., may be as follows: IC50 values <50 = strong binder, 50 ≤ .. ≤500 = medium, 500 ≤ .. ≤ 5000 nM = low binder, >5000 random binder. In general, the IC50 (half maximal inhibitory concentration) is considered a measure used in pharmacology and also to classify and quantify the binding strength of peptides to HLA, by indicating how much of a particular molecule is needed to inhibit the binding of a reference peptide to a HLA molecule by 50% (e.g., by disrupting the binding between the reference peptide and the HLA molecule, e.g., how much of the respective molecule (peptide of interest) is needed to inhibit 50% of the binding of a competitive reference target). In embodiments NetMHCpan is used in step e. for predicting the binding of peptides to HLA molecules, which preferably predicts how well a peptide binds to HLA class I molecules, and preferable is pan-allelic, and/or also functions on novel alleles. In embodiments, in step e., the used tool outputs IC₅₀ and/or %Rank to classify binding strength of a respective peptide of interest to a HLA molecule.

Herein, the term 'reference peptide' preferably refers to one or more peptides that were comprised within the experimental determination of the interaction/ binding data of the respective at least one protein of interest. In other words, a reference peptide is preferably a peptide for which interaction / binding data to a respective protein of interest is provided (as input to the computational model(s)), that has been determined before, preferably experimentally, and/or for which structural and/or sequence data is provided as input to the computational model(s). Hence, in embodiments for one or more reference peptides structural and/or sequence data and/or interaction / binding data to a respective protein of interest is provided as input to the computational model(s), preferably for training purposes of the computational model(s).

In general, the Major Histocompatibility complex (MHC) system is known in human as the human leukocyte antigen (HLA). The group of MHC/HLA genes in humans is located on chromosome 6 and encodes genes associated with immune system function in humans, such as cell-surface antigen-presenting proteins. The major HLA antigens are considered to be essential elements for immune function. Human leukocyte antigen molecules (HLA molecules) are proteins found on the surface of most cells in the human body that play a crucial role in the immune system. HLA molecules aid the immune system to distinguish self from non-self proteins, while specifically presenting protein fragments (peptides, antigens) to cells of the immune system, such as T cells. HLA molecules may be grouped into two major classes, namely HLA Class I and II. HLA antigens that correspond to MHC class I (HLA class I molecules) may be grouped into HLA-A, HLA-B or HLA-C 2. HLA class I molecules are found on all nucleated cells and present intracellular peptides (e.g., viral proteins) and are known to interact with CD8+ T cells (cytotoxic T cells). HLA Class II molecules (corresponding to MHC class II molecules) may be grouped into HLA-DP, HLA-DQ, HLA-DR. HLA Class II molecules are found mostly on immune cells (e.g., macrophages, dendritic cells, and B cells) and present extracellular peptides (e.g., such as bacterial proteins) and are known to interact with CD4+ T cells (helper T cells).

As used herein a HLA-peptide (pHLA) complex refers preferably to a molecular structure formed by a peptide binding to a HLA molecule. Commonly, such pHLA complexes are displayed on the surface of cells to be recognized by T cells of the immune system via their T-cell receptor (TCR).

In embodiments, the *in vitro* binding or interaction (high throughput) screening used for generating training data comprises a certain pHLA set, that is used for testing the binding or interaction of a protein of interest thereto. In embodiments, one marker characterizing a pHLA set used in said binding screen may be its mutation distance to a target peptide sequence, wherein a distance of 1 means that the peptide (sequence) differs by 1 mutation (e.g., 1 amino acid) from the target peptide (sequence).

Herein, a 'target peptide sequence' or 'target sequence' is preferably an amino acid sequence that is considered to be comprised within, or to be the (ideal/optimal) target binding sequence of a protein of interest, such as e.g., a target epitope of an antibody (fragment), a receptor (e.g., TCR) or DARPIN.

As used herein, an immunoassay or affinity-assay may refer in embodiments to a biochemical method employed to detect the presence or quantify the concentration of a macromolecule or polypeptide in a solution or to determine its binding strength and/or kinetics, or affinity to a protein of interest, e.g., utilizing said protein of interest, e.g., an antibody, antibody fragment, DARPin, receptor or immunoglobulin. Exemplary types of immunoassays include luminescence immunoassays (LIA), radioimmunoassays (RIA), chemiluminescence and fluorescence immunoassays, enzyme immunoassays (EIA), enzyme-linked immunosorbent assays (ELISA), luminescence-based bead arrays, magnetic bead-based arrays, protein microarray assays, rapid test formats, and rare cryptate assays. The term immunoassay encompasses a range of techniques, including, but not limited to, enzyme immunoassays (EIA) such as enzyme multiplied immunoassay technique (EMIT), enzyme-linked immunosorbent assays (ELISA), antigen capture ELISA, sandwich ELISA, IgM antibody capture ELISA (MAC ELISA), and microparticle enzyme immunoassays (MEIA); capillary electrophoresis immunoassays (CEIA); radioimmunoassays (RIA); immunoradiometric assays (IRMA); fluorescence polarization immunoassays (FPIA); lateral flow assays (LFA); turbidimetric assays; and chemiluminescence assays (CL) and label free binding kinetic techniques, such as SCORE, SPR and BLI.

Herein, the method may comprise or use computational means and/or computational models, such as one or more machine learning (ML)-based model(s), one or more artificial intelligence (AI) and/or one or more neural network (NN) or any combination thereof.

In general, machine learning comprises the field of supervised learning, namely the learning of a computational model or AI from labeled data, comprising linear regression, logistic regression, decision trees, random forests, k-nearest neighbors (k-NN), support vector machines (SVM), gradient boosting, as well as the field unsupervised learning, namely the learning of patterns from unlabeled data, comprising k-means clustering, hierarchical clustering, principal component analysis (PCA), DBSCAN and autoencoders. Another filed of machine learning is semi-supervised learning that comprises a mix of labeled and unlabeled data, such as contrastive learning or contrastive learning objectives. Machine learning commonly further comprises the field of reinforcement learning (RL).

In embodiments herein the one or more machine learning (ML)-based model(s) or one or more artificial intelligence (AI) comprise or consist of one or more neural networks (NN), and optionally further (implemented) algorithms, Al or computational models. In embodiments the computational means referred to herein, e.g., as 'AI' or 'at least one machine learning model' may comprise numerous and diverse computational means that may work together and/or may be used in a computational pipeline when performing the present method.

In general, the term 'neural networks' comprises artificial neural network (ANN; in contrast to biological neural networks), such as convolutional neural networks (CNN), recurrent neural networks (RNN), transformer networks, generative adversarial networks (GAN), feedforward neural networks (FNN), spiking neural networks (SNN), multilayer perceptron (MLP) and autoencoders (encoder/decoder networks).

Commonly, generative adversarial networks (GAN) comprise two networks, comprising a generator and a discriminator. Commonly, autoencoders may be used for dimensionality reduction, denoising and anomaly detection. Commonly a FNN may be used for (simple) classification/regression. Recurrent neural networks (RNN) are commonly used to handle sequences, comprising LSTM (long short-term memory), and GRU (gated recurrent unit).

The present Al or ML-model is preferably able to perform tasks, such as regression modelling comprising predicting a value, such as Kd value, classification of data comprising predicting a class, such as binder or non-binder, and/or generation of data, comprising generating or predicting a new protein structure.

In embodiments of the present method different machine learning (ML) models may be used, and preferably trained on experimental binding/interaction and/or structure and/or sequence data of peptides (preferably comprised within pHLA complexes) and proteins of interest. Non-limiting and purely exemplary models comprise ESM and AntiBerty models, which are capable of predicting protein structures and interaction / binding based on learnings from training data.

In one non-limiting example, input data, comprising sequence data of reference peptides and/or proteins(s) of interest and/or *in vitro* binding/interaction data of the protein(s) of interest and the reference peptides is embedded (comprising the conversion of amino acid sequences to numerical representations) by ML-models, preferably pre-trained ML-models, such as ESM and/or AntiBerty. Subsequently the binding of a protein of interest may be predicted by the trained ML-model(s), e.g., based on sequence data of a peptide on interest provided to the ML-Model(s).

In embodiments of the present invention various applicable embedding models could be used interchangeably, for example any substitution matrix like BLOSUM62, or any pretrained neural-networks that can handle sequences of amino acids, such as ESM2 (Evolutionary Scale Modelling). In embodiments, an embedding model may be employed and trained on experimental data and be incorporated as part of the ML- or Al model used for the final prediction in method step e. Similarly, in embodiments the ML- or AI model may comprise or consist of at least one neural-network or gradient boosted model and may have a variety of architectures such as a multi-layer perceptron (MLP), Transformer, or convolutional neural network CNN. The inventors have successfully used a number of different variants of computational models, such that the present method is considered to not depend on any specific computational implementation.

In embodiments a protein of interest may be a receptor, an antibody, or an antibody fragment or any equivalent of an antibody or antibody fragment, or a designed ankyrin repeat protein (DARPin) or any comparable structure capable of specifically recognizing and binding antigens.

As utilized herein, the term "antibody" generally refers to a protein consisting of one or more polypeptide chains primarily encoded by immunoglobulin genes or their fragments. The term "antibody" is intended to encompass "antibody fragments" and any equivalents thereof as well. The term "antibody" is intended to encompass immunoglobulins or may be used interchangeably with "immunoglobulin" herein.

In general, the immunoglobulin genes encompass the constant region genes for kappa, lambda, alpha, gamma, delta, epsilon, and mu, in addition to a diverse array of immunoglobulin variable region genes. Light chains are classified as either kappa or lambda, while heavy chains are categorized as gamma, mu, alpha, delta, or epsilon, which define the immunoglobulin isotypes IgG, IgM, IgA, IgD, and IgE, respectively. The fundamental structural unit of an antibody (immunoglobulin) is typically a tetramer or dimer. Each tetramer consists of two identical pairs of polypeptide chains, with each pair comprising one "light" (L) chain (~ 25 kDa) and one "heavy" (H) chain (~ 50-70 kDa). The N-terminal region of each chain defines a variable region, generally spanning approximately 100 to 110 amino acids, which is primarily responsible for antigen recognition. The terms "variable heavy chain" and "variable light chain" refer to the variable regions of the heavy and light chains, respectively. Optionally, the antibody or its immunologically active fragment may be chemically conjugated or expressed as a fusion protein with other proteins.

The term "specific binding", e.g., of a protein of interest, antibody, antibody fragment, receptor or DARPin, is intended to be interpreted as via a skilled person, who is clearly aware of different experimental procedures that may be used to test binding and binding specificity. While some degree of cross-reaction or background binding may occur in many protein-protein interactions, such occurrences do not undermine the "specificity" of the binding between an antibody and its epitope. Additionally, the term "directed against" is relevant in the context of "specificity" when describing the interaction between an antibody, antibody fragment, receptor or DARPin and its target epitope.

Antibodies of the present invention include, but are not limited to, polyclonal, monoclonal, bispecific, human, or chimeric antibodies, single-domain antibodies (e.g., VHH fragments derived from nanobodies), single-variable fragments (ssFv), single-chain variable fragments (scFv), Fab fragments, F(ab')2 fragments, fragments produced via Fab expression libraries, anti-idiotypic antibodies, epitope-binding fragments, or any combination thereof, provided that they retain the original binding properties. Additionally, mini-antibodies and multivalent antibodies, such as diabodies, tetravalent antibodies, triabodies, and peptabodies, may also be employed in the methods described herein.

DARPins (Designed Ankyrin Repeat Proteins) are commonly considered a class of engineered proteins comprising ankyrin repeat domains, that constitute structural motifs consisting of tandem repeats, e.g., of ~ 33 amino acids. DARPins may be designed to bind with high specificity to a wide range of targets, including proteins, peptides, and other biomolecules, making them versatile tools in molecular biology, diagnostics, and therapeutics.

The immunoglobulin molecules referenced herein can belong to any class (i.e., IgG, IgE, IgM, IgD, or IgA) or subclass of immunoglobulins. Accordingly, the term "antibody," as used in this context, also encompasses antibodies and antibody fragments that are either modified from whole antibodies or synthesized de novo using recombinant DNA technology.

A "variable region" of an antibody refers herein to the variable region of either the antibody light chain or the antibody heavy chain, individually or in combination. The variable regions of both the light and heavy chains comprise four framework regions (FRs) interspersed with three complementarity-determining regions (CDRs), also known as hypervariable regions. As used herein, a CDR may refer to CDRs defined by any suitable method. In embodiments, variations of variable region across different species, may be used and/or tested in the context of the present invention.

Herein a 'solid support' may be a microfluidic chip or device, microarray, glass plate, plastic plate or multi-well plate or any surface suitable for immobilizing a reference peptide, pHLA complex or protein of interest. In embodiments solid support of a certain kind may also be a streptavidin-coated solid support, or equivalents allowing for protein or peptide immobilization, optionally for covalent or non-covalent, permanent or transient immobilization.

Herein the term 'physiochemical properties' preferably comprises characteristics of amino acids influencing a proteins structure, such as hydrogen bonding, hydrophobicity, or charge. Inherently, the physiochemical properties of a peptide or protein are determined by the corresponding properties of its amino acids (sequence).

In general and in embodiments of the present invention, common physiochemical properties comprise, but are not limited to, molecular weight, LogP, LogD, pKa, aqueous solubility, hydrogen bond donors, hydrogen bond acceptors, topological polar surface area (TPSA), number of rotatable bonds, molecular volume, polarizability, refractivity, permeability, plasma protein binding, stability (chemical/metabolic), isoelectric point (pl), lipophilicity, charge at physiological pH.

Herein, an optimal, high or even highest possible binding affinity (e.g., of all possible amino acid sequences, aa-motifs, peptides or proteins) refers to the binding affinity of a peptide to a protein of interest or vice versa, which is indicative of a positive binding of said peptide to a protein of interest or vice versa, namely a certain physicochemical affinity leading to their binding/interaction, which is preferably lower (the lower the KD value, the stronger the binding/ interaction) than the binding affinity of the peptide or protein of interest to other proteins of interest or peptides, respectively.

In embodiments, the sequence and/or structural data may comprise information on the amino acid sequence, the nucleic acid sequence encoding a respective peptide or protein, the three-dimensional (3D) structure, the two-dimensional (2D) structure, the SMILES string, the InChl, the InChlKey, the chemical structure, any other biochemical property of a biomolecule, and/or other molecule features. In embodiments, the sequence and/or structural data may be / have been determined experimentally or may be / have been predicted by one or more computational model, such as a ML-model or AI.

The term 'human immunopeptidome' describes in general the entirety of all peptides that are being presented on HLA molecules on the surface of cells. These peptides originate from the digestion of cellular proteins. The immunopeptidome is a central part for the immune system. It provides it with the possibility to distinguish between foreign and self or healthy and sick.

### FIGURES

The invention is further described by the disclosed figures. These are not intended to limit the scope of the invention but represent preferred embodiments of aspects of the invention provided for greater illustration of the invention described herein.
**Figure 1****:** Entire workflow of an embodiment of the present invention for predicting suitable peptides that may be presented to a protein of interest in the context of a pHLA complex in a screening. The flowchart illustrates the interaction between different steps, or 'modules' of an embodiments of the present method. In a specific embodiments the present method comprises two modes, a human and non-human. In embodiments, the human mode comprises in return two sub modes: off-target identification (solid line) and peptides annotation (dotted line). Optionally, the non-human mode may comprise off-target identification mode only. In the figure, the module's name reflects what the preferred or main task of the module.
   For example, in one embodiment of the present invention comprising the target sequence of a protein of interest (e.g., target motif, amino acid sequence), suitable HLA alleles/molecules for presenting peptides and/or information regarding both that may be obtained from databases or online resources, such as scientific databases, scientific publications and/or patent publications. Such data may be obtained by computational means, such as one or more ML-models, algorithms or other computational workflows. B. The core steps of an embodiment of the present invention comprising computational method steps, such as the "FindTopes" step (e.g., equivalent method step c.), wherein off targets are identified based on sequence or structural similarity of potential target peptides for a protein of interest, e.g., combining BLASTp with other online databases or tools. Another computational method step may be the "MutaTopes" step (e.g., equivalent method step d./e.), wherein a mutation analysis for peptides is performed and tailored to a specific population of mutated sequences with a AF> 0.1, e.g., with a certain mutation distance to a known target sequence of a protein of interest. Another step is the "PrediTopes" step (e.g., equivalent method step. f.), comprising leveraging internal data and/or the IEDB, and/or comporting an *in silico* prediction of peptide binding, processing and elution, e.g., predicting affinity between peptides and MHC / HLA alleles/molecules. The "AnnoTopes" step (e.g., equivalent method g.) may comprise the analysis, determination and/or prediction of data regarding the gene expression of potential peptides in different tissues, aiding the prediction of off-target or target binding in different tissue, in case the protein of interest is a therapeutic or similar molecule. The "AnnoTopes" step may further or alternatively comprise the consideration of or prediction of biophysical features extracted from sequence and 3D structure. The step may be used for or comprise predicting gene expression tissue-wise, biophysical features of peptides, biophysical features. The "ViralTopes" step may comprise the identification of off-targets of a protein of interest in viruses and other non human genomes that may be hosted by humans. C. The ranking phase of embodiments of the present invention may comprise a bi-feature formula-based ranking comprising, e.g., the analysis and/or consideration of targeted features of respective potential peptides and their clustering and ranking based on these. Therefrom a ranking of likely targets and/or off-target peptides may be obtained to prioritize the most promising peptides for the downstream analysis. Multivariable (predicting one response based on multiple features) embedding-based ranking may also be performed, e.g., comprising the selection of features (only relevant pr predictive features may be considered), dimension reduction (comprising e.g., PCA, t-SNE and/or UMAP) and/or clustering and ranking using k-mean and/or e.g., HDBSCAN. Finally, an output is generated comprising preferably structured reports and a summary of potential peptides for the downstream interaction screening, which may be used for aiding an informed decision on the most suitable and promising peptides for such an interaction screening for a protein of interest.
**Figure 2****:** A. The Figure shows one embodiment of the method step also termed 'FindTopes' (e.g., equivalent method step c.) wherein off targets are identified based on sequence or structural similarity of potential target peptides for a protein of interest, e.g., combining BLAST with other online databases or tools. In the shown exemplary workflow of a FindTopes work- and data flow, after the results are merged and filtered, the module creates *yaml* file for the following modules and preferably a report at the end. B. The Figure shows one embodiment of the mutation logic, which may be employed by the 'MutaTopes' method step (e.g., equivalent method d./e.), wherein a mutation analysis for peptides is performed and tailored to a specific population of mutated sequences, e.g., with a AF> 0.1, e.g., with a certain mutation distance to a known target sequence of a protein of interest. In the scheme, the same genomic SNPs map to different protein coordinates across different isoforms, enabling differential peptide overlap patterns. Here, the resulting peptides would have the same sequence, if the SNPs alternative allele was identical across both isoforms, yet preferably the final list of peptides would be isoform specific.

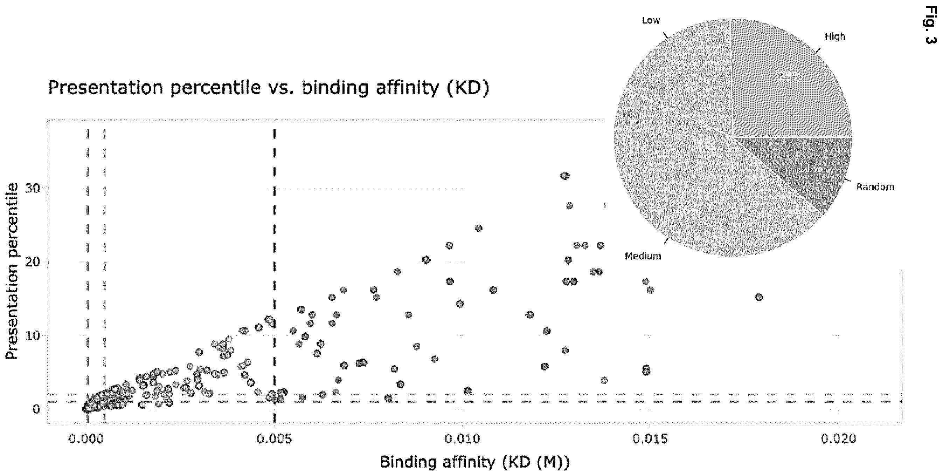

**Figure 3****:** scatter plot of affinity vs. presentation percentile, since these two measurements do not correlate linearly, peptides that are considered high binders and highly presented are of particular interest (lower left corner, the lower the better); pie chart showing peptides categories by binding affinity.

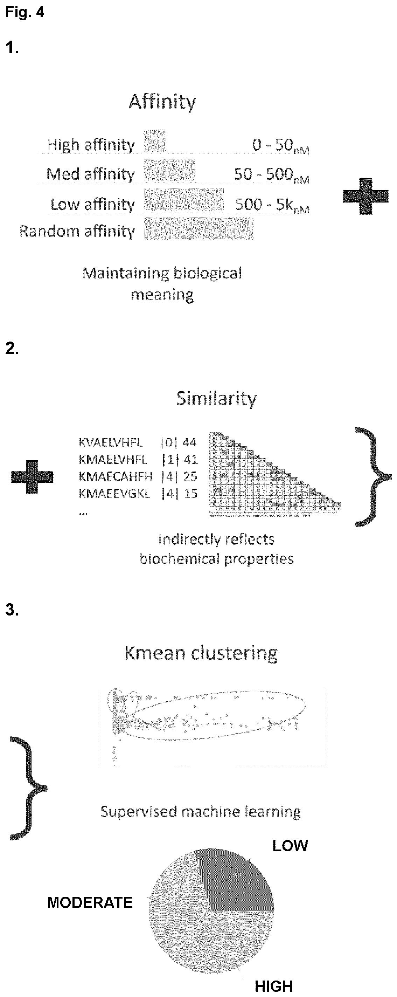

**Figure 4****:** Bi-features clustering. In this embodiment, the score prioritizes off-targets based on similarity to the target, using BLOSUM62 substitution matrix, and its affinity to HLA-allele, IC50. The higher the score is, the higher the risk is, i.e., highly ranked peptides are more likely to be relevant off-targets and therefore should be included in downstream analysis. 1. The peptides are ranked according to their affinity (maintaining biological meaning), then according to their sequence similarity (2) and finally Kmean clustering is performed (SEQ ID NO: 1-4) (3) and the peptides are ranked in 'high', 'medium' and 'low' when k =3.

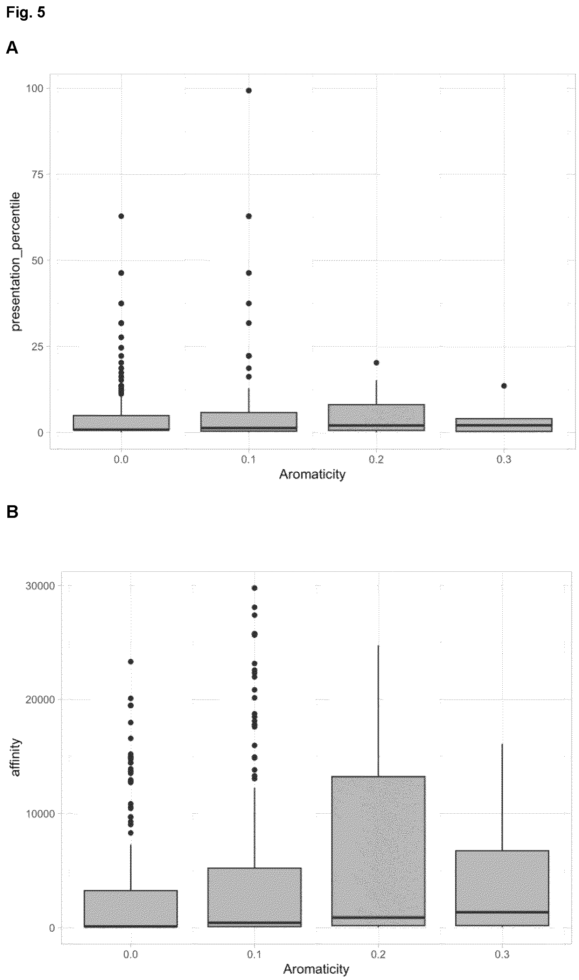

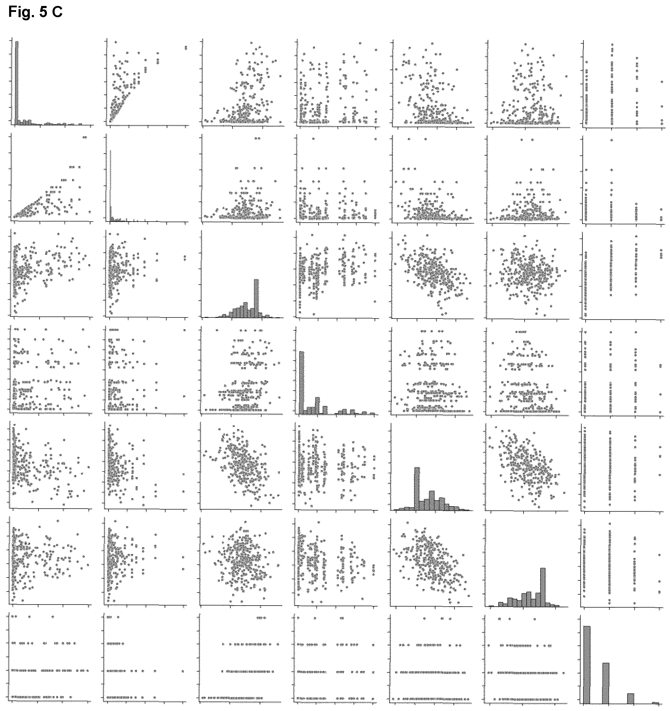

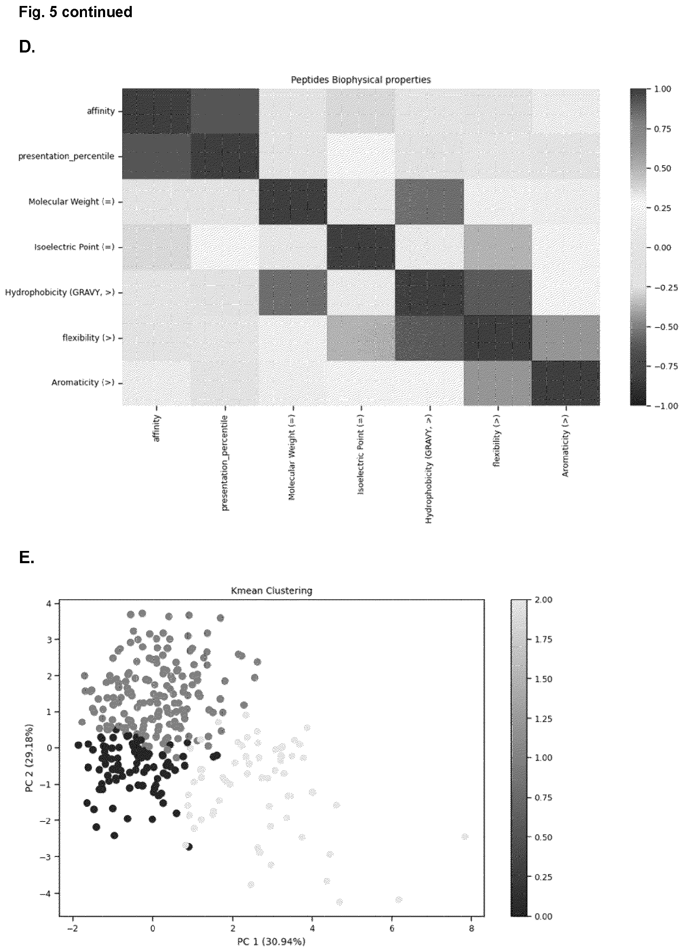

**Figure 5****:** Biophysical property correlation according to embodiments of the present method. A. presentation percentile (Y-axis) vs. aromaticity (X-axis). B. Affinity (Y-axis) vs. aromaticity (X-axis). C. The shown correlation of scatter plots and histograms includes in the present example (Y-axis, from top to bottom and X-axis from left to right): affinity, presentation percentile, molecular weight, isoelectric point, hydrophobicity, flexibility, and aromaticity. D. Peptides biophysical properties (same Y- and X-axis as C.). E. K-means clustering, PC2 (Y-axis), PC1 (X-axis).

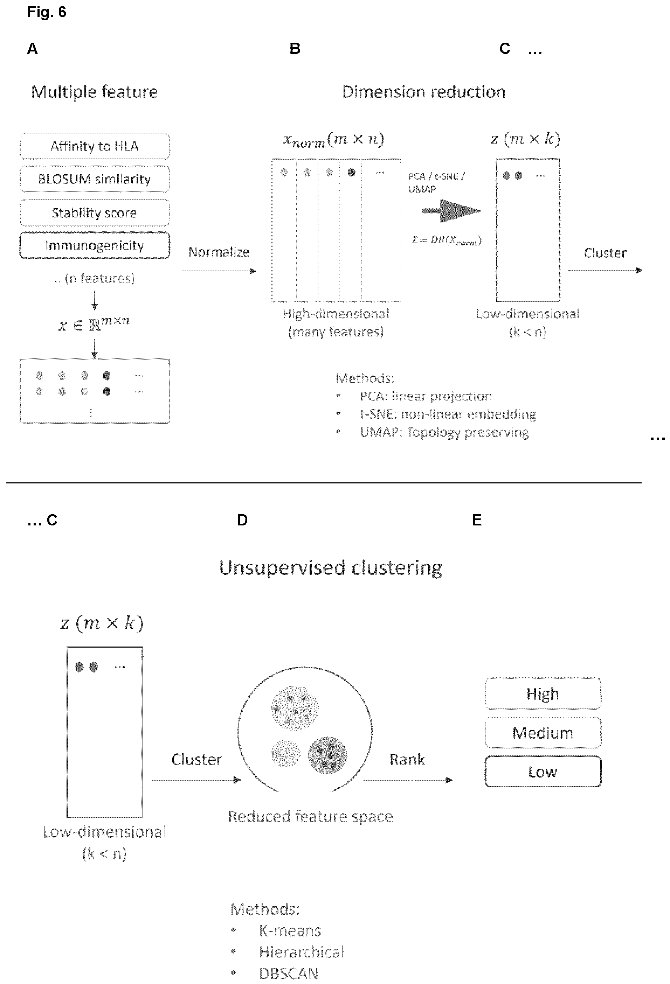

**Figure 6****:** Multi-features score. The workflow is depicted in two lanes, Steps A-E depicting consecutive steps of an embodiments of a scoring approach according to the present method, namely A. selection/consideration of multiple features, B. followed by a first high dimensional reduction and a second (C) low dimensional reduction. D unsupervised clustering to reduce feature space and outputting/generating/providing a final score, e.g., ranked in 'high', 'medium' and 'low'.

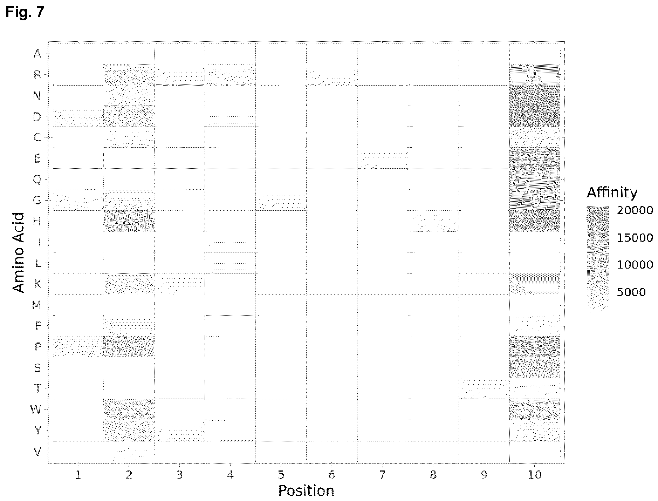

**Figure 7****:** Results of a subsequent *in vitro* interaction screening ('x-scan') with the peptides predicted according to the present method. The heatmap shows predicted affinity vs. amino acid substitution at each position. In this ex-ample the peptide's length is 10. The heatmap shows clear association between positions 2 and 10 with the binding affinity. Substitutions at these positions show drop in the affinity (large value), indicating that these are the two positions that are relevant for MHC complex to form.

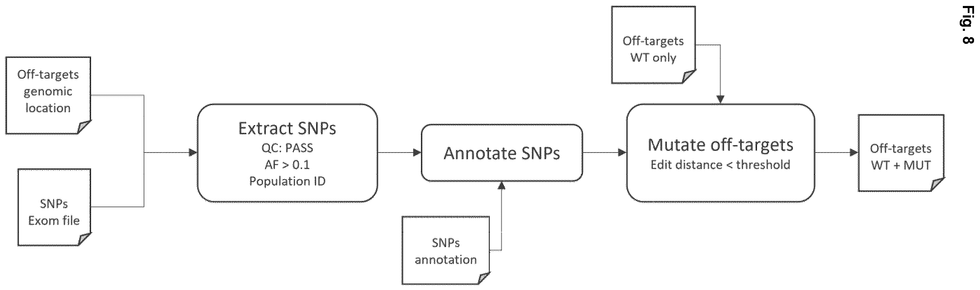

**Figure 8****:** Workflow of the SNP integration according to one embodiment of the present method (MutaTopes; equivalent method step d./e.).

### EXAMPLES

The invention is further described by the disclosed examples. These are not intended to limit the scope of the invention but represent preferred embodiments of aspects of the invention provided for greater illustration of the invention described herein. Any of the features described in the following may, in embodiments, also be considered separately or in combination with each other.

### Example 1

An exemplary workflow of the presently tested embodiment is shown in Figure 1.

In this example an input comprised the target sequence of a protein of interest (e.g., target motif, amino acid sequence), suitable HLA alleles/molecules for presenting reference peptides and/or information regarding both that may be obtained from databases or online resources, such as scientific databases, scientific publications and/or patent publications. Such data was obtained by computational means, such as one or more ML-models, algorithms or other computational workflows. During the core steps of the tested embodiment the computational method steps comprised the steps of "FindTopes" step (e.g., equivalent method c.), wherein off targets are identified based on sequence or structural similarity of potential target (reference) peptides for a protein of interest, e.g., combining BLASTp with other online databases or tools.

Another computational method step was the "MutaTopes" step (e.g., equivalent method step d./e.), wherein a mutation analysis for reference peptides is performed and tailored to a specific population of mutated sequences with a AF> 0.1, e.g., with a certain mutation distance to a known target sequence of a protein of interest. Subsequently the "PrediTopes" step (e.g., equivalent method step f.) comprised leveraging internal data and/or the IEDB, and/or comporting an in silico prediction of peptide binding, processing and elution, e.g., predicting affinity between peptides and MHC / HLA alleles.

The subsequent "AnnoTopes" step (e.g., equivalent method step g.) comprised the analysis, determination and/or prediction of data regarding the gene expression of potential reference peptides in different tissues, aiding the prediction of off-target or target binding in different tissue, in case the protein of interest is a therapeutic or similar molecule. The "AnnoTopes" step could optionally further or alternatively comprise the consideration of, or prediction of biophysical features extracted from sequence and 3D structure. The step may be used for or comprise predicting gene expression tissue-wise, biophysical features of peptides, biophysical features.

The "ViralTopes" comprised the identification of off-targets of a protein of interest in viruses that may be hosted by humans.

During the ranking phase a bi-feature formula-based ranking comprising, e.g., the analysis and/or consideration of targeted features of respective potential reference peptides and their clustering and ranking based on these was performed. Therefrom a ranking of likely targets and/or off-target reference peptides was obtained to prioritize the most promising peptides for the downstream analysis.

Multivariable embedding-based ranking was also performed comprising the selection of features (only relevant pr predictive features may be considered), dimension reduction (comprising e.g., PCA, t-SNE and/or UMAP) and/or clustering and ranking using k-mean and/or HDBSCAN.

Finally, an output was generated comprising a structured report and a summary of potential reference peptides for the downstream interaction screening, which could subsequently be used for aiding an informed decision on the most suitable and promising reference peptides for such an interaction screening for a protein of interest.

### Example 2

In the present exemplary embodiments of the method according to the invention, input information is loaded and compiled from (online) databases and datasets in a first step (e.g., equivalent method step b.). In one embodiment, such step may be achieved with the assistance of computational means, such as an "DBfetch" algorithm, which obtains and provides all required input data to the present method and the ML-models employed, preferably it also insures error-free cross-referencing and/or data sanity.

For example, such a computational means, e.g., "DBfetch" was used, operated as follows. Input data was obtained (downloaded) from (online) data resources/databases, followed by optional quality checks, a filtering and/or reformatting of input data. In the present example, these operations included individual workflows, and/or standalone scripts. Herein, the step of obtaining and providing input data (by DBfetch) was executed once, it may alternatively by executed when updating the resources either to the latest or approved version.

In the present example, the following sources for input data were used:
The public datasets used were the following (it is possible to use one or more thereof in different embodiments):
- **UNIPROT:** protein sequences (canonical and isoforms), protein annotation according to uniprot.
- **HPA:** gene expression of normal and blood samples. The data set used is the "HPA RNA transcript data" which consists of 40 different tissues.
- **GTEx:** as second resource for gene expression in normal tissue. Herein, GTEx version 8 was used.
- **Gnomad:** all SNPs reported in the coding part of the gene (exon). The SNPs are filtered by their quality (pass) and the population. Version 2.1.1 is used that was left over to GRCh38 ensuring matching GTEx version.
- **Ensembl:** annotation binary files; these files are important for SNPs annotating tools to function. Version 110 -> GRCh38 is used as it is compatible with the used Gnomad version.
- HLA-related DBs: -> PEPREP (in-house database, e.g., comprising mass spectrometry data) for hosting curated MHC complexes and propriety data.

Subsequently, the input data was analyzed regarding possible peptides with a sequence similarity to the target peptide of a protein of interest. This step (method step c.) may also be termed 'FindTopes' herein (see also Fig. 2 A).

In a second step (a second 'module') potential off-target peptides (peptides of interest) were identified using different approaches to increase the coverage of potential off-targets, wherein the selection and analysis was based on sequence similarity to the target.

The tool 'BLASTp' was used in two modes: short and long. The substitution matrix for the short mode, blastp-short, was BLOSUM62 while for the long mode PAM250. PAM matrices are generally considered lenient as they are more permissive to substitutions (mismatches are less penalized) and therefore more suitable for distance homologs. By switching to PAM250, BLASTp can retrieve long distanced peptides that could be missed by BLOSUME matrices. E-value was also changed to 1e⁻⁶ threshold to ensure safe statistics, reduced noise and thus correct results. Subsequently, one list was constructed from the unique joined results of both modes.

Additionally, PEPMatch was used to identify more peptides of interest (potential off-targets). PEPMatch is a deterministic tool that identifies similar peptides using k-mer approach. Our results show that relatively similar peptides were easily and jointly identified by both tools. However, the tools identified different sets on the opposite side of the spectrum. BLASTp reported highly similar peptides that PEPMatch missed, while PEPMatch identified highly divergent peptides that BLASTp didn't. These findings confirmed that the two tools complement each other in the search for all potential off-targets (peptides of interest), with their distinct algorithms making our overall search more comprehensive and thorough.

Subsequently, the following cutoffs were applied to narrow down the total identified peptides of interest (potential off-targets):
- Mismatch cutoff: on average 50-60% dissimilarity rate/edit distance compared to the target is al-lowed.
- Length cutoff: all off-targets must have the same length as the target.
- No gaps (in alignment) are allowed.

This all served the goal of finding as much and as heterogenous off-target peptides (peptides of interest) of a protein of interest as possible. Once a 'final' list was created, each protein of interest (potential off-target) was mapped to its genomic and proteomic location coordinates.

As a third step, the genomic coordinates of each off-target that were extracted in the second step ('FindTopes'; method step c.) were used to select and extract the SNPs found within the respective genomic coordinates (see e.g., Fig. 2B). The SNPs were filtered according to the population, quality and/or allele frequency (~> 0.1, preferably >0.1 ± 10, 15 or 25 %). The quality filter was chosen, such that only experimentally and/or validated reported SNPs were included, while predicted SNPs or SNPs partially passing the calling filters were excluded from the downstream analysis.

Once the SNPs were extracted, they were fully annotated using VEP, thus their proteomic positions and/or impact could be determined. Every SNP was first analyzed whether its position overlapped the wildtype off-target, coming from the same gene and same isoform (see Fig. 2B). The amino acid at the position of the SNP was then substituted by the SNP to retrieve the mutated peptide in comparison to the wildtype. By repeating this procedure with every SNP and every off-target, the list of the potential off-targets could be increased, since it included the off-targets identified for the natural (wild type) form of the peptide and those potentially resulting from a mutation. Finally, the edit distance of the newly added peptides was calculated, keeping only those peptides within the dissimilarity rate, while the remaining were excluding.

The resulted peptides list was isoform specific, as both peptide and SNP locations depended on the formed isoform, as their coordinates could be different despite having the same sequence. All results were reported as output, to enable a more detailed downstream analysis. At this point, the identified peptides/off-targets comprises basic information comprising sequence, gene-protein mapping, transcript-isoform mapping, edit distance, wildtype/mutated annotation and/or the position on the protein.

Subsequently, for the assessment of the risk of a peptide of interest being an off-target of a protein of interest, the probability of a peptide of interest being presented on a cell and/or the affinity of a respective peptide of interest to a HLA-allele of interest is determined ('PrediTopes' step; method step f.). A presentation score and/or the affinity to a HLA-allele of interest were obtained using NETMHCpan and used to classify the peptide of interest (see Figure 3). Furthermore, in this module, the context of peptide of interest was explored via MHC databases. Two main resources were used, IEDB and PEPREP, in conjunction with an in-house database that covered experimentally detected/verified MHC complexes. Information, such as the experimentally identified HLA-allele, and/or the relevant tissue in which the complex may be found and/or the binding strength, plus optionally further relevant information was considered and provided as an output, e.g., comprising a representation as table and/or graphical output. In this example, this further relevant information was obtained on two levels, the off-target sequence and the protein ID and was later used in 'derisking' the off-target.

In a further step ('AnnoTopes'; method step g.), all annotations were integrated with each other, along with gene expression (both on gene and transcript levels) allowing scientists to make further decisions and adding more selection criteria. In addition to the annotation, peptide ranking/risk scores were also integrated.

First a so called 'bi-features score' was determined, which prioritizes peptides of interest as off-targets based on similarity to the target, e.g., using BLOSUM62 substitution matrix, and/or its affinity to HLA-allele, e.g., indicated by the respective IC50 (see Figure 4). The higher the bi-features score is, the higher the risk is, i.e., highly ranked peptides of interest are more likely to be relevant off-targets of a protein of interest and therefore should be considered during/for in downstream analysis.

The score function (S) is defined as $S \left(a, b\right) = \frac{f \left(a\right) + g \left(b\right)}{2}$, where: $f \left(a\right) = \left\{1, 0.66, 0.33, 0\right\} for a ∈ \left\{\left[0, 50\right), \left[50, 500\right), \left[500, 5000\right), \left[5000, ∞\right)\right\}$ $g \left(b\right) = \frac{b - {b}_{min}}{{b}_{max} - {b}_{min}}$

The final peptide classification is then estimated using supervised machine learning method, k-means with 3 clusters (e.g., as default clustering mode): ${peptide}_{rank} = {kmeans}_{3} \left(S\right) \to \left\{Low, Moderate, High\right\}$

Subsequently, a 'multi-features score' was determined, which may be considered an approach suitable for handling high-dimensional feature spaces, thereby allowing for a better integration of a peptide's (of interest) physiochemical features and other features contributing to a peptide's biological context, thereby likely also to their relevance as off-targets (see Figure 6). First, the feature matrix was constructed $X = \left[{x}^{1},{x}^{2},…,{x}_{n}\right] ∈ {ℝ}^{mxn}$, where *m* m was the total number of peptides, *n* total number of features (affinity, BLOSUM, stability, expression, etc.) and *xᵢ* represented a features vector.

The features were first normalized: $X _ norm = \left(X-μ\right) / σ or X _ norm = \left(X-X_min\right) / \left(X_max-X_min\right) .$

Then a dimension reduction technique was used: $Z = DR \left(X_norm\right) ∈ {ℝ}^{mxk} where k < n and DR ∈ \left\{PCA,t-SNE,UMAP\right\} .$

Subsequently, an unsupervised clustering was performed: $\left.C=Clustering\left(Z, {k}_{clusters}\right)\right| C ∈ \left\{K-means,hierarchical,DBSCAN\right\} .$

Finally, the ranking is defined as *Rank*(*peptideᵢ*) *= f*(*clusterᵢ, distance_to centroidᵢ*)*.*

Subsequently, in one further step, non-human genomes were considered (step also termed 'ViralTopes'), herein by identifying peptides of interest, which may constitute off-targets, in pathogen and/or virus species, which are preferably only hosted by humans, e.g., HIV. In this step, the identification of peptides of interest likely being off-targets of a protein of interest, was performed similar as in the above-described 'FindTopes' step (method step c.) and by applying the same cutoffs and criteria (length, edit distance, etc.; see above).

Finally, to identify likely HLA-binding motifs within the target peptide sequence, the binding motif the target sequence of a protein of interest was modified/mutated one amino acid at the time. Due to the number of different amino acids, the total number of modified target sequence versions would be L×19. The affinity to the HLA-allele was then predicted *in silico* using NETMHCpan for every mutated target motif variation. The results then revealed the potential binding motif/sequence of the target peptide with a HLA-molecule/allele, i.e., how the complex will be formed and consequently which part of the sequence that could be eventually exposed to the protein of interest in an *in vitro* screening (see Figure 7).

### Example 3

The following architecture of a method according to the present invention constitutes a specific embodiment, wherein each method step 1-8, or 'module 1-8', may be considered a specific, non-limiting example of the features of the present invention. However, in embodiments of any of the features or steps described above, the following features may be considered in combination, or separately in a non-limiting manner.

In one exemplary embodiment of the present method it may comprise two modes, an off-target identification (1.1.) and a peptide annotation (1.2.) mode. In embodiments, the steps or modules that are used may differ according to the mode.

### 1.1 Off-target identification

### (see also solid lines in Figure 1)

*Input Parameters:*
   - HLA allotype specifications (standard nomenclature)
   - Target sequence of any length (typically 8-15 amino acid length)
   - Mismatch threshold
   - Population
   - Allele frequency threshold
*Processing Workflow:*
   - Execute module FindTopes to identify off-targets in human genome
   - Execute module MutaTopes to integrate SNPs
   - Predict in-silico measurements in module PrediTopes and
   - Add experimental annotation according to MHC databases (IEDB + PEPREP (in-house MHC resource))
   - Execute module AnnoTopes to aggregate and add more annotations, then risk-rank the off-targets
   - Output ranked and fully annotated off-targets (binding predictions with experimental validation status and gene expression)
   - Execute module ViraTopes for non-human off-targets (optional).
   - Execute module xscan (*in silico* target peptide mutation) to identify HLA-allele binding motifs with the target
*Output Data Structure:*
   - Off-target sequences with full annotation
   - Binding affinity predictions (IC50 values)
   - Presentation score and percentile
   - Classification categories (high/medium/low)
   - Experimental validation flags
   - Tissue-specific context data
   - Gene expression
   - Synthetic peptides list

### 1.2 Peptides annotation

### (see also Dotted lines in Figure 1)

*Input Parameters:*
   - HLA allotype specifications (standard nomenclature)
   - Target sequence of any length (typically 8-15 amino acid length)
   - Peptide list to be annotated and classified
*Processing Workflow:*
   - Execute module Annotator to annotate peptides (gene-protein)
   - Predict in-silico measurements in module PrediTopes and
   - Add experimental annotation according to MHC databases (IEDB + PEPREP (in-house MHC re-source))
   - Execute module AnnoTopes to aggregate and add more annotations, then risk-rank the off-targets
   - Output ranked and fully annotated off-targets (binding predictions with experimental validation status and gene expression)
*Output Data Structure:*
   - Peptides sequences with full annotation
   - Binding affinity predictions (IC50 values)
   - Presentation score and percentile
   - Classification categories (high/medium/low)
   - Experimental validation flags
   - Tissue-specific context data
   - Gene expression

### 2 DBfetch (equivalent method step b.)

*Input Parameters:*
   - Database identifiers and API endpoints
   - Authentication credentials and access protocols
   - Database-specific filtering thresholds:
      a. SNP database: exon regions only
      b. Tools specific input
      c. Expression database: tissues with available samples-meta info
      d. Protein database:
         i. Experimentally validated protein sequences (flag = SP (Swiss-Prot))
         ii. Isoforms mapping availability
         iii. Viruses and bacteria protein sequences that hosted by human
   - Update frequency parameters (monthly/yearly)
   - Data quality validation criteria
   - Version control
   - Cross-reference mapping capability
*Processing Workflow:*
   1. Database query engine:
      a. Execute parallel API calls to configured databases
      b. Implement retry logic and error handling
      c. Apply database-specific filters
   2. Data standardization pipeline:
      a. Convert heterogeneous formats in unified schemas when possible
      b. Unify identifiers across database sources
      c. Resolve cross-reference mapping and other conflicts
   3. Quality control processor
      a. Validate data integrity and completeness
      b. Highlight inconsistencies, missing annotation and excluded data
      c. Generate quality metrics and processing summaries
   4. Integration and indexing engine
*Output Data Structure:*
   *Example of standardized database:*
   1. Protein sequence repository:
      a. FASTA filtered sequences
      b. Protein and isoforms sequences
      c. Cross-reference mapping (UniProt, Ensembl, etc.)
   2. Population genetics dataset:
      a. Indexed SNP variant files (VCF format)
      b. Population frequencies
      c. Quality metrics
      d. SNP annotations files (genomic coordinates)
   3. Gene expression matrix:
      a. Tissue-specific expression levels (TPM/nTPM/pTPM)
         - Cell-level
      b. Gene and transcript level
   4. Cross-reference mapping tables:
      a. Gene ID to protein ID mappings
      b. Transcript ID to isoform ID mappings
      c. Genomic coordinates to gene positions
      d. Gene type annotation source
      e. Version tracking

### 3 FindTopes (equivalent method step c.)

*Parallel Search Architecture:*
   1. BLASTp dual-mode search:
      a. Short mode: BLOSUM62 matrix, E-value 1e-6
      b. Long mode: PAM250 matrix, E-value 1e-6
   2. PEPMatch k-mer search:
      a. Deterministic k-mer decomposition algorithm
      b. Distance-based similarity scoring
   3. Result integration process
      a. Duplicates removal algorithm

   - Cross-validation between the different methods
*Processing Workflow:*
   - Target peptide preprocessing:
   - Validate input sequence format and protein info
   - Generate search parameters for each algorithm
   - Dual BLASTp search:
   - Short: Execute BLOSUM62-based search
   - Conservative substitution scoring matrix
   - Detects highly similar peptides that could be missed by PEPMatch
   - Statistical significance threshold: E-value ≤ 1e-6
   - Long: Execute PAM250-based search
   - Lenient substitution matrix for distant homologs
   - Enhanced sensitivity for divergent sequences
   - Extended evolutionary distance detection
   - PEPMatch k-mer Analysis:
   - Decompose target into overlapping k-mers (k=2)
   - Execute deterministic similarity matching
   - Identify divergent peptides missed by BLASTp algorithms
   - Result integration and deduplication:
   - Merge results from all search algorithms
   - Remove duplicate identifications across methods
   - Generate unified candidate off-target list
   - Multi-criteria filtering:
   - Sequence dissimilarity constraints (50-60% maximum dissimilarity)
   - Exact length matching
   - Relaxed length
   - Exclude gap-containing peptides
   - Genomic coordinate mapping:
   - Map peptide sequences to protein database entries
   - Extract genomic coordinates and chromosomal positions using cross-reference tables
   - Generate cross-reference annotations
*Technical specifications:*
   *Input Parameters:*
      - Target peptide sequence (typically anything between 8-15 amino acids)
      - Reference protein databases (UniProt)
      - Configurable similarity thresholds (default: 50-60% dissimilarity)
      - Gap penalty configurations (default: gaps disabled)
   *Algorithm-Specific Settings:*
      - BLASTp Short Mode:
      - Substitution matrix: BLOSUM62
      - Word size: 3
      - Gap penalties: disabled
      - BLASTp Long Mode:
      - Substitution matrix: PAM250
      - Word size: 2
      - PEPMatch Configuration:
      - k-mer size: configurable (default: 2)
      - Distance metric: Hamming
   *Output data structure:*
      - Comprehensive Off-Target Dataset:
      - Peptide Sequences: Amino acid sequences
      - Edit distance scores
      - Source Protein Information :
         - Protein identifiers and annotations
      - Genomic Coordinates:
         - Chromosomal positions (GRCh38/hg38)
         - Gene symbol mappings
         - Transcript/isoform variant information
      - Algorithm Provenance:
      - Detection method flags (BLASTp-short/long, PEPMatch)
      - Cross-validation status
   *Specific technical features:*
      1. Dual-Matrix BLASTp Implementation:
         a. Parallel execution of BLOSUM62 and PAM250 substitution matrices
         b. Captures both close evolutionary relationships (BLOSUM62) and distant homologs (PAM250)
         c. Addresses coverage limitations of single-matrix approaches
      2. Dual-Algorithm Integration for Off-Target Discovery:
         a. Systematic combination of BLASTp and PEPMatch for comprehensive off-target identification
         b. Complementary detection capabilities
         c. Cross-algorithm validation ensuring comprehensive peptide coverage
      3. Complementary Coverage Optimization:
         a. Based on empirical analysis of detection patterns
         b. Systematic approach leverages probabilistic and deterministic methods

### 4 MutaTopes (equivalent method step d./e.)

Architecture: MutaTopes integrates SNPs by generating off-target peptide variants through systematic integration of single nucleotide polymorphism that are associated with selected population.

### Processing Workflow:

1. Genomic-based coordinate SNP extraction:
   a. Query population-specific SNP databases
   b. Extract SNPs using off-target genomic coordinates
   c. Apply multi-criteria filtering pipeline:
      - Quality validation filter (experimentally validated SNPs only)
      - Allele frequency threshold (≥ 0.1 population frequency)
      - Coding SNPs
      - Exclude computationally predicted variants without experimental validation
2. Variant effect prediction and annotation:
   a. Execute VEP (Variant Effect Predictor) annotation pipeline
   b. Determine and extract proteomic coordinate mappings
   c. Determine alternative alleles
   d. Calculate variant impact scores and functional consequences
   e. Generate transcript-specific variant annotations
3. Isoform-Specific coordinate:
   a. Cross-reference SNP positions with off-target peptide coordinates
   b. Validate gene identity and isoform compatibility
   c. Ensure isoform-level coordinate consistency
   d. Filter for overlapping genomic positions
4. Generate off-target peptide variant:
   a. Systematic amino acid substitution at SNP positions:
      - Extract wildtype amino acid at SNP coordinate
      - Substitute with SNP-derived amino acid variant (alternative allele)
      - Generate mutated off-target peptide sequence
      - Maintain peptide length consistency
   b. Apply substitution logic across all validated SNP-peptide pairs
5. Sequence Similarity Validation:
   a. Calculate edit distance for newly generated mutant peptides
   b. Apply dissimilarity rate thresholds (consistent with FindTopes)
   c. Exclude peptides with high dissimilarity (> threshold)
   d. Maintain sequence diversity (remove duplicates due to mutations)
6. Comprehensive Dataset Assembly:
   a. Integrate wildtype and mutant peptide variants
   b. Generate isoform-specific result sets
   c. Maintain complete tracking for variant origin (isoform, allele, position and impact)
   (see also Figure 2B: MutaTopes, SNPs integration algorithm and logic)

### Technical specifications:

*Input Parameters:*
   - Off-target genomic coordinates from FindTopes module
   - Population identifiers (gnomAD project)
   - Quality control thresholds:
   - Allele frequency minimum: ≥ 0.1
   - Quality validation: experimental evidence required
   - VEP configuration parameters
   - Sequence similarity constraints (inherited from FindTopes)
*SNP Database Integration:*
   - Primary sources: gnomAD
   - Population stratification categories
   - Quality score thresholds (FILTER = "PASS")
   - Functional annotation requirements
*Isoform Processing:*
   - Transcript annotation (Ensembl)
   - Coordinate system: GRCh38/hg38 genomic coordinates
   - Cross-reference validation: gene symbols, transcript IDs, uniprot & isoforms

### Output data structure:

*Population-Specific Off- Target Dataset:*
   - Peptide variant information:
   - Wildtype peptide sequences with genomic coordinates and transcript id
   - SNP-derived mutant off-target sequences
   - Updated edit distance scores
   - Genomic annotation data:
   - Gene identifiers and protein mappings
   - Transcript and isoform specifications
   - Chromosomal coordinates
   - VEP functional impact predictions
   - Population genetics metadata:
   - SNP identifiers (rs numbers) and allele frequencies
   - Quality validation status and evidence sources
   - Provenance Tracking:
   - Wildtype vs. mutant classification flags
   - SNP-to-peptide mapping relationships
   - Isoform-specific coordinate systems
*Noval technical features:*
   1. Population-stratified off-Target variant generation:
      - First systematic integration of population genetics with off-target prediction
      - Automated SNP-to-peptide coordinate mapping across isoforms
      - Population-specific risk assessment for drug off-target effects
   2. Genomic coordinate-based SNP integration:
      - Direct coordinate mapping from off-target identification to variant databases
      - Isoform-specific coordinate validation and cross-referencing
      - Systematic quality filtering for experimental validation
   3. Comprehensive variant effect integration:
      - VEP-based functional annotation of off-target variants
      - Multi-level coordinate validation (genomic → transcript → protein-isoform)
      - Edit distance preservation across wildtype and mutant peptide sets
   4. Population-specific drug safety framework:
      - Integration of allele frequency data with off-target predictions
      - Population stratification for personalized risk assessment
      - Systematic expansion of off-target space through genetic variation

### 5 PrediTopes (equivalent method step f.)

Architecture: PrediTopes integrates *in-silico* predictions (binding and presentation) and is considered a validation module to distinguish high-priority off-targets from low-risk candidates through systematic integration of predictive algorithms with experimental evidence databases, IEDB and the inhouse curated database PEPREP.
*Input Parameters:*
   - HLA allotype specifications (standard nomenclature)
   - Peptide sequences (8-15 amino acid length)
   - Peptide's protein-id
*Processing Workflow:*
   1. HLA prediction:
      a. Execute NetMHCpan predictions with the selected HLA allotype
      b. Generate in-silico prediction profile
      c. Classify off-targets by binding, presentation strength
   2. Experimental evidence cross-validation:
      a. Query IEDB (Immune Epitope Database) for experimental data:
         i. Search for identical peptide sequence
         ii. Retrieve experimental information (protein, HLA binding info, tissue, diseases)
         iii. Validate prediction against experimental
         iv. Repeat on protein level
      b. Query PEPREP for additional validation:
         i. Access internal experimental data
         ii. Retrieve all relative experimental information
         iii. Obtain binding information

         - Cross-reference between the two resources
   3. Contextual risk stratification:
      a. Integrate prediction and experimental evidence
         i. Apply weighting metric to prediction based on experimental info
         ii. Mark off-targets with confirmed experimental info
         iii. Mark the discrepancies between predictions and experiments
         iv. Generate confidence scores for the prediction
      b. Apply biological context analysis:
         i. Assess tissue/disease relevance
         ii. Evaluate clinical information if available
         iii. Generate first risk classification
   4. Urgent off-target identification approach:
      a. Generate actionable risk categories:
         i. Urgent: high binding + high presented + experimental evidence
         ii. Important: strong prediction (binding + presentation) + partial experimental evidence (e.g., different HLA allotype)
         iii. Study/assess: moderate predictions + limited experimental support (e.g., only protein was found)
         iv. Low priority: weak predictions + no experimental confirmation
*Output Data Structure:*
   - Binding affinity predictions (IC50 values)
   - Presentation score and percentile
   - Classification categories (strong/weak/non-binder)
   - Experimental validation flags
   - Tissue-specific context data
*Specific technical features:*
   1. Prediction-validation integration intelligence
   2. Contextual risk intelligence system:
      a. Multi-database context evaluation
      b. Experimental evidence
      c. Experimental evidence weighting
   3. Urgent off-target classification framework
   4. Dual-database validation architecture:
      a. Public database integration for broad and community-proven experimental coverage
      b. Proprietary database integration (PEPREP) for specialized validation
      c. Enhance confidence through cross-database validation
      d. Comprehensive experimental evidence aggregation and analysis

### 6 AnnoTopes (equivalent method step q.)

Architecture: AnnoTopes comprises bi- and multi-dimensional scoring configured to integrate biological annotations, gene expression, and physiochemical properties for comprehensive off-target risk stratification.
*Processing Workflow:*
   1. Comprehensive annotation integration:
      - Integrate multi-source biological annotations
      - Import gene expression data (gene-level and transcript-level)
      - Cross-reference tissue-specific expression profiles
      - Generate unified annotation datasets for downstream scoring
   2. Physicochemical calculation:
      - Calculate sequence-based physicochemical:
      - Gravy scores
      - Molecular weight and amino acid composition
      - Isoelectric point and charge distribution
      - Secondary structure propensities
      - Aromatic content and flexibility measures
      - Estimate features correlation matrices
      - Apply correlation analysis to identify informative features
      - Filter redundant or non-informative physicochemical features
   3. Bi-Features risk scoring algorithm:
      - Calculate BLOSUM62-based sequence similarity scores
      - Integrate HLA binding affinity data (IC50 values)
      - Apply feature normalization
      - Apply dual-parameter scoring function for risk assessment
      - Execute supervised clustering for clustering
      - Generate preliminary risk classifications
   4. Multi-Features advanced scoring:
      - Construct high-dimensional feature matrices:
         o Filter physicochemical properties
         o Sequence based metrics
         o HLA prediction metrics
         o Gene expression data
      - Apply feature normalization and dimensionality reduction
      - Execute unsupervised clustering for clustering
      - Generate risk classifications
   5. Used machine learning-based classification:
      - Implement supervised k-means clustering (default: 3-cluster)
      - Implement unsupervised methods (dimension reduction: PCA, t-SNE, UMAP; clustering: kmeans, hierarchal, DBSCAN)
      - Generate risk categories based on multi-dimensional analysis
      - Apply ranking algorithms for final prioritization
*Technical specifications:*
   *Bi-features scoring algorithm:*
      *Mathematical Framework:*
      - Score Function: S(peptide) = f(BLOSUM62, HLA_affinity)
      - Where:
         - BLOSUM62: sequence alignment score against the target
         - HLA_affinity: IC50 binding strength
         - Higher scores indicate increased off-target risk
   *Scoring Parameters:*
      - BLOSUM62 matrix: Indirectly reflects biochemical properties
      - HLA binding threshold: IC50 values (<50, 50 ≤ .. ≤500, 500 ≤ .. ≤ 5000 nM)
      - Risk classification: supervised k-means clustering (default: k=3)
      - Output categories: high-risk, medium-risk, low-risk classifications
   (see also Figure 5C-E: bi-features score)
*Physicochemical features process:*
   *Property calculation pipeline:*
   - Sequence-Based Calculations:
   - GRAVY (Grand Average of Hydropathy) scores
   - Molecular weight and amino acid frequencies
   - Isoelectric point (pl) calculations
   - Aromaticity indices and flexibility measures
   - Secondary structure prediction scores
*Feature selection logic:*
   - Correlation matrix analysis:
   - Calculate pairwise correlations between all physicochemical features
   - Apply correlation thresholds (typically r > 0.8 for redundancy detection)
   - Identify informative vs. redundant feature combinations
   - Feature filtering:
   - Remove highly correlated redundant features
   - Retain physicochemical properties with unique information content
   - Optimize feature set for downstream machine learning algorithms
   (see also Figure 6: multi-features processing)

### Multi-features scoring algorithm:

*Features matrix:*
   - Dimensionality: m × n matrix where:
   - m = total number of off-target peptides
   - n = total number of integrated features
   - Feature vector components:
   - HLA binding affinity scores
   - BLOSUM62 sequence similarity metrics
   - Physicochemical properties (stability, charge, hydrophobicity, etc.)
   - Gene expression levels (tissue-specific)
   - Gene expression levels (cell-specific)
   - Immunogenicity score
   - Additional biological context features
*Features processing pipeline:*
   - Feature Normalization: Z-score standardization across feature dimensions
   - Dimensionality Reduction: Principal Component Analysis (PCA) or equivalent
   - Clustering Algorithm: Unsupervised machine learning classification (UMAP) or other
   - Ranking Function: Multi-dimensional risk score calculation
*Mathematical implementation:*
   - Feature Matrix: X(m×n) with normalized values
   - Dimensionality Reduction: X' = PCA(X) where X' has reduced dimensions
   - Clustering: C = cluster(X') using unsupervised methods
   - Final Ranking: R = rank(C) based on cluster characteristics and risk profiles
   (see also Figure 6: multi-features score)
*Input parameters:*
   - Off-target peptide sequences
   - Physicochemical features
   - Annotation including:
   - HLA binding affinity predictions (IC50 values)
   - HLA presentation prediction
   - MHC database relevance
   - Gene expression datasets (RNA-seq)
   - Tissue-specific expression profiles
*Output data structure:*
   *Comprehensive risk assessment list:*
      - Off-targets risk scores:
      - Bi-features scores with BLOSUM62 and HLA binding affinity
      - Multi-features scores with high-dimensional integration
      - Machine learning-based risk classifications
      - Ranking priorities for downstream analysis
      - Annotation data:
      - Integrated biological annotations
      - Gene and transcript expression levels
      - Tissue-specific context information
      - Physicochemical property profiles
      - Classification results:
      - Supervised clustering assignments (3-cluster system)
      - Risk category classifications (high/medium/low)
      - Confidence scores and statistical metrics
      - Feature contribution analysis
   *Specific technical features:*
      1. Dual-scoring architecture:
         - Bi-features algorithm for preliminary assessment
         - Multi-features system for comprehensive high-dimensional analysis
         - Scalable scoring methodology adaptable to different analytical contexts
      *2. Multi-dimensional risk integration:*
         - Systematic integration of sequence similarity, binding affinity, and physicochemical proper-ties
         - Machine learning-based pattern recognition in high-dimensional feature spaces
         - Automated dimensionality reduction for complex biological datasets
      *3. Machine learning classification:*
         - K-means clustering with configurable cluster numbers (default k=3)
         - Risk stratification based on multi-parameter analysis
         - Statistical validation of clustering assignments and risk categories
      *4. Comprehensive Biological Context Integration:*
         - Gene and transcript-level expression integration
         - Tissue-specific annotation cross-referencing
         - Multi-source biological annotation consolidation

### 7 ViralTopes

Architecture: ViralTopes is a cross-species screening module for identifying potential crossreactive off-targets within human-hosted microorganism proteomes.

### Processing Workflow:

*1. Human-hosted pathogen database curation:*
   - Query pathogen taxonomy databases for human-associated species
   - Filter organism databases by host specificity criteria:
      ∘ Viruses: human-tropic strains and variants
      ∘ Bacteria: human microbiome and pathogenic species
      ∘ Parasites: human-infective lifecycle stages
      ∘ Fungi: human-associated opportunistic pathogens
   - Maintain updated pathogen proteome repositories
   - Maintain version information
*2. Cross-species similarity search:*
   - Execute identical algorithm framework as FindTopes module:
      ∘ BLASTp dual-mode implementation (BLOSUM62 + PAM250)
      ∘ PEPMatch k-mer analysis for deterministic matching
      ∘ Parallel algorithm execution with result integration
   - Apply consistent filtering criteria:
      ∘ Length matching requirements (exact target length)
      ∘ Edit distance constraints (default 50-60% dissimilarity)
      ∘ Gap elimination (no gap-containing alignments)
*3. Pathogen-specific result processing:*
   - Cross-reference identified peptides with pathogen annotations
   - Validate organism classification and human-host relationships
   - Maintain organism provenance and taxonomic information
*4. Human cross-reactivity assessment:*
   - Evaluate potential for molecular mimicry with human targets
   - Assess immunological cross-reactivity risks
   - Generate pathogen-specific risk classifications

### Technical specifications:

*Input Parameters:*
   - Target peptide sequences from primary analysis
   - Human-hosted pathogen proteome databases
   - Organism filtering criteria:
      ∘ Host specificity requirements (human-associated only)
      ∘ Pathogen classification categories (virus, bacteria, parasite, fungi)
   - Similarity search parameters (inherited from FindTopes)
*Algorithm configuration:*
   - Search methodology: identical to FindTopes dual-algorithm approach
   - Filtering criteria: consistent length and similarity constraints
   - Quality control: gap elimination and sequence validation
*Output data structure:*
   *Cross-species off-target list:*
      - Pathogen off-target matches:
         ∘ Sequence with edit distance scores
         ∘ Organism source identification and taxonomic classification
         ∘ Pathogen-specific annotations and functional categories if available

      - Organism metadata:
         ∘ Pathogen taxonomy and classification information
         ∘ Strain and variant information
         ∘ Database provenance and version tracking
   *Clinical and research application*
      - Vaccine development
      - Drug safety assessment
      - Immunotherapy design
      - Infectious disease research
      - Biodefense applications

### 8 xscan

Architecture: XScan module systematically substitutes amino acid to determine position-specific HLA binding preferences and identify critical residues for peptide-MHC complex formation and later antibody binding.
*Processing Workflow:*
   1. Target Sequence Preparation:
      - Validate input target peptide sequence length and composition
      - Generate amino acid position mapping for systematic substitution
      - Initialize substitution matrix for all possible amino acid variants
   2. Systematic single-position substitution:
      - Execute comprehensive amino acid substitution protocol:
         ∘ For each position i in target sequence (1 to L, where L = sequence length)
         ∘ Generate 19 alternative amino acid substitutions at position i
         ∘ Create modified peptide variants maintaining original length
         ∘ Total variants generated: 19 × L modified sequences
   3. HLA Binding prediction for peptide variants:
      - Execute NetMHCpan predictions for the complete set:
      - Process original target sequence as reference
      - Predict HLA binding affinity for all 19×L substitution variants
      - Create comprehensive binding landscape visualization
   4. Binding motif analysis and characterization:
      - Analyze position-specific binding contributions:
      - Compare variant binding affinities to original sequence
      - Identify critical residues for HLA binding maintenance
      - Determine permissive vs. restrictive amino acid positions
      - Calculate position-specific binding preference matrices
   5. Structural and immunological interpretation:
      - Generate binding motif profiles for peptide-HLA complex formation
      - Identify potential antibody exposure sites based on binding analysis
      - Predict structural implications of amino acid substitutions
      - Create comprehensive binding landscape visualization
*Technical specifications:*
   *Input Parameters:*
      - Target peptide sequence (8-15 amino acid length)
      - HLA allotype specifications (standard nomenclature)
   *Substitution protocol:*
      - Systematic substitution: single position modification per variant
      - Total variant generation: 19 substitutions × target sequence length
      - HLA prediction: using NetMHCpan affinity and other in silico prediction
   *Output data structure:*
      *Comprehensive binding motif peptide variant list:*
      - Peptide variant predictions:
      - Prediction values for all 19×L substitution variants
      - Comparative analysis against original target sequence
      - Position-specific analysis:
      - Binding contribution scores for each amino acid position
      - Critical residue identification (anchor positions vs. auxiliary residues)
      - Amino acid preference matrices per position
      - Permissive vs. restrictive position classifications
      - Binding motif characterization:
      - HLA binding motif profiles and consensus sequences
      - Structural implications of amino acid substitutions
      - Predicted antibody accessibility regions
      - Binding landscape visualization data
      - Immunological prediction:
      - Potential T-cell epitope regions based on binding analysis
      - Antibody exposure probability estimates
      - Cross-reactivity risk assessments with variant peptides

## Claims

1. A computer-implemented method for predicting at least one peptide of interest binding to a protein of interest comprising one or more of the following steps:
a. providing sequence information of at least one target peptide known to bind to a protein of interest,
b. providing human protein sequence information,
c. determining at least one peptide of interest by comparing the sequence of the at least one target peptide to the human protein sequence information provided in b., wherein the at least one peptide of interest comprises a sequence that has at least 50% sequence identity to the sequence of the least one target peptide, and optionally the same length ± 25 % as the least one target peptide,
d. providing information on one or more single nucleotide polymorphism (SNP) present within the human genome, at least comprising the genomic location (genomic coordinates) and the polymorph amino acid of said one or more SNP,
e. analyzing and annotating the one or more SNP regarding its potential position within a peptide of interest, comprising (i) determining the presence of the one or more SNP within the sequence of a peptide of interest and, if present, (ii) additionally generating the sequence of a respective peptide of interest comprising said one or more SNP, preferably wherein the analysis and annotation is isoform specific,
f. predicting the binding affinity (e.g., as IC50) of the at least one peptide of interest determined in c., and optionally in e., to a HLA-molecule, thereby predicting the likelihood of the at least one peptide of interest being presented in a HLA-complex on the surface of a cell,
g. combining the data obtained in steps. a.-f. and ranking, optionally using a ranking score, the at least one peptide of interest regarding the likelihood of binding to the protein of interest, thereby predicting the likelihood of the at least one peptide of interest binding to a protein of interest.

2. The method according to claim 1, wherein the method further comprises, analogous to steps b. and c. of claim 1., providing protein sequence information of human-hosted viruses, preferably obtained from one or more (online) databases comprising sequence information of proteins expressed by human-hosted viruses, preferably comprising information on the respective amino acid sequence of said proteins, and optionally the genes encoding said proteins, and/or their nucleic acid sequence, and
determining at least one viral peptide of interest by comparing the sequence of the at least one target peptide to the provided viral protein sequence information, wherein the at least one viral peptide of interest comprises a sequence that has at least 50% sequence identity to the sequence of the least one target peptide, and optionally the same length ± 25 % as the least one target peptide, and
wherein in step g. of claim 1 the data on viral peptide of interest is combined with the data obtained in steps. a.-f. and ranked accordingly.

3. The method according to claim 1 or 2, wherein the method further comprises h. selecting at least one of the highest ranking peptide(s) of interest predicted in g. and performing an *in vitro* and/or *in silico* interaction screening to validate the predicted binding of the at least one peptide of interest to the protein of interest determined according to the method of claim 1 or 2.

4. The method according to any one of the preceding claims, wherein any one of steps a.-g. are performed by employing computational means, preferably comprising one or more (trained) machine learning (ML)-based model or artificial intelligence (AI).

5. The method according to claim 3, wherein the at least one peptide of interest is presented in the *in vitro* and/or *in silico* interaction screening within a HLA-peptide (pHLA) complex.

6. The method according to any one of the preceding claims, wherein step e. of claim 1 comprises filtering the in (ii) additionally generated peptides of interest sequence(s) for a sequence identity to the sequence of the least one target peptide of ≥ 50%.

7. The method according to any one of the preceding claims, wherein the list of the peptide(s) of interest generated in step g. of claim 1 is complemented by a list of (*in silico*) mutated target peptide sequences, wherein each mutated target peptide sequence comprises one amino acid substitution over the wild type sequence of the at least one target peptide,
preferably wherein each consecutive amino acid of the entire sequence length of the at least one reference peptide has been mutated accordingly, and wherein each mutated target peptide sequence is provided with a binding affinity (e.g., as IC50) to a HLA molecule, preferably predicted according to step f. of claim 1.

8. The method according to claim 5, wherein the *in vitro* interaction screening to validate the predicted binding of the one or more peptides of interest to the protein of interest comprises the following steps:
i. providing at least one peptide of interest, predicted in step g. to (likely) bind to the protein of interest,
ii. supplying HLA-proteins, preferably biotinylated HLA-proteins, to the at least one peptide of interest and allowing the HLA -proteins to bind to the at least one peptide of interest, thereby forming pHLA complexes,
iii. immobilizing the formed pHLA complexes on a solid support, preferably on a streptavidin-coated solid support,
iv. contacting the immobilized pHLA complexes with the protein of interest,
v. determining binding data of the individual pHLA complexes with the protein of interest.

9. The method according to claim 8, wherein the binding data determined in the interaction screening comprises the binding strength, preferably comprising the dissociation constant (KD), of the protein of interest to at least one peptide of interest.

10. The method according to claim 8 or 9, wherein the data obtained from the interaction screening is subsequently used to determine and/or compare potential peptides of interest constituting a off-target binding of the protein of interest.

11. The method according to any one of claims 8-10, wherein the protein of interest is a receptor, antibody, antibody fragment or equivalent thereof, wherein the equivalent of a receptor, an antibody or antibody fragment thereof is any biomolecule capable of recognizing and/or specifically binding antigens and/or capable of (specifically) binding to pHLA complexes, such as, e.g., DARPINs.

12. The method according to the preceding claim, wherein the protein of interest is a TCR-like antibody (TCRm) or fragment thereof, or a T cell receptor or fragment or equivalent thereof.

13. The method according to any one of the preceding claims, wherein the human protein sequence information provided in step b. is obtained from one or more (online) database comprising sequence information of proteins expressed in humans, preferably comprising information on the respective amino acid sequence of said proteins, and optionally the genes encoding said proteins, their genomic location (genomic coordinates) and/or their nucleic acid sequence.

14. The method according to any one of the preceding claims, wherein in step g. combining the data obtained in steps. a.-f. and ranking the at least one peptide of interest regarding the likelihood of binding to the protein of interest considers at least the sequence similarity to the at least one target peptide and/or the binding affinity to a HLA-molecule determined in f., and optionally further considers the physiochemical properties of the at least one peptide of interest
